# EUROPEAN PATENT APPLICATION

(11) **EP 4 825 844 A1**
(43) Date of publication of application: **21.10.2026**
(21) Application number: 24902919.0
(22) Date of filing: 12.12.2024
(51) Int. Cl.: C07D 239/42, A61K 31/505, A61P 29/00

(54) **NAV1.8 INHIBITOR COMPOUND, SALT THEREOF, POLYMORPH THEREOF, AND USE THEREOF**

(30) Priority: 13.12.2023 CN 202311723384; 04.12.2024 CN 202411777862
(71) Applicant: Yichang Humanwell Pharmaceutical Co., Ltd., Yichang, Hubei 443005 (CN); Wuhan Humanwell Innovative Drug Research and Development Center Limited Company, Wuhan, Hubei 430075 (CN)
(72) Inventor: ZHANG, Xuejun, Wuhan, Hubei 430075 (CN); LI, Jinping, Wuhan, Hubei 430075 (CN); ZONG, Qiao, Wuhan, Hubei 430075 (CN); XIA, Qingfeng, Wuhan, Hubei 430075 (CN); CHEN, Haomin, Wuhan, Hubei 430075 (CN); SONG, Xiaobo, Wuhan, Hubei 430075 (CN); LIU, Lifei, Wuhan, Hubei 430075 (CN); YANG, Jun, Wuhan, Hubei 430075 (CN); LI, Li'e, Wuhan, Hubei 430075 (CN)
(74) Representative: Gille Hrabal Partnerschaftsgesellschaft mbB Patentanwälte
(86) International application number: PCT/CN2024/138893
(87) International publication number: WO 2025/124502

(57) **Abstract**

The present invention relates to a Navl.8 inhibitor compound, a salt thereof, a polymorph thereof, and the use thereof. The present invention provides a salt of a compound of formula (I), a compound of formula (I) and a crystal form of a salt thereof, which have good medicinal properties. The structure of the formula (I) is as follows:

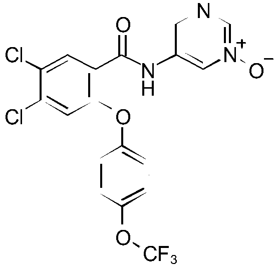

## Description

This application claims the benefit of priority from the earlier applications filed by the applicant:
with the China National Intellectual Property Administration on December 13, 2023, application number 202311723384.5, entitled "A Nav1.8 inhibitor compound and its salt, polymorph and use"; and
with the China National Intellectual Property Administration on December 4, 2024, application number 202411777862.5, entitled "A Nav1.8 inhibitor compound and its salt, polymorph and use";
the entire contents of which earlier applications are incorporated herein by reference.

### TECHNICAL FIELD

The present disclosure belongs to the field of pharmaceuticals, and relates to a Nav1.8 inhibitor compound, a salt thereof, a polymorph thereof, and preparation methods and applications thereof.

### BACKGROUND

Pain is "an unpleasant sensory and emotional experience associated with, or resembling that associated with, actual or potential tissue damage," and it is a subjective experience. Pain can serve as a warning signal, alerting the organism to potential danger, and plays an indispensable protective role in the normal life activities of the organism. Simultaneously, pain is also a common clinical symptom. After the external stimulus causing the pain disappears, intense or persistent pain can lead to physiological dysfunction, severely affecting the quality of life of the living organism. According to statistics, approximately one-fifth of the world's population suffers from moderate to severe chronic pain. The global analgesic market was about 360 billion US dollars in 2018 and is projected to reach 560 billion US dollars by 2023. Among these, the market for acute moderate-to-severe pain is expected to grow steadily at a compound annual growth rate of 2.5%, while the future market for chronic pain will grow at a compound annual growth rate of about 18%. Chronic pain is the primary driving force for the sustained growth of the global pain market over the next decade.

Pain originates from nociceptors in the peripheral nervous system. These are free nerve endings widely distributed in the skin, muscles, joints, and visceral tissues throughout the body. They convert perceived thermal, mechanical, or chemical stimuli into nerve impulses (action potentials), which are then transmitted via afferent nerve fibers to their cell bodies located in the dorsal root ganglia (DRG), and ultimately to higher neural centers, resulting in the sensation of pain. The generation and conduction of action potentials in neurons depend on voltage-gated sodium channels (NaV) on the cell membrane. When the cell membrane depolarizes, sodium channels activate, open, and cause an influx of sodium ions, leading to further depolarization of the cell membrane and the generation of an action potential. Therefore, inhibiting abnormal sodium channel activity contributes to the treatment and alleviation of pain.

Human sodium channels are a class of transmembrane ion channel proteins composed of α subunits with a molecular weight of 260 kDa and β subunits with molecular weights of 30-40 kDa. Based on the differences in α subunits, they can be classified into nine subtypes, namely Nav1.1 to Nav1.9. Nav1.5, Nav1.8, and Nav1.9 are tetrodotoxin (TTX)-insensitive sodium channels. Nav1.5 is primarily found in cardiomyocytes, while Nav1.8 and Nav1.9 are present in the peripheral nervous system. Nav1.8 is a crucial ion channel involved in chronic pain, atrial fibrillation, and Budd-Chiari syndrome, and serves as a highly selective therapeutic target for pain management.

The Nav1.8-encoding gene is SCN10A, located on the human chromosome 3p21-22 region, primarily encoding the α subunit. Studies have found that the homology between human and rat Nav1.8 genes is as high as 93%. Nav1.8 is predominantly found in trigeminal ganglion neurons and DRG neurons and exhibits electrophysiological characteristics of slow inactivation and rapid recovery. In neurons expressing Nav1.8, the upstroke of the action potential is primarily constituted by the Nav1.8 current. In neuropathic pain models, nerve injury leads to an increased expression level of Nav1.8 in axons and neuronal cell bodies. The use of Nav1.8 antisense oligonucleotides can significantly alleviate pain while reducing Nav1.8 expression. After injecting carrageenan into the rat paw, the expression of Nav1.8 in DRG neurons increases. Nav1.8 knockout mice fail to exhibit normal visceral inflammatory pain. Function-gain mutations in the human Nav1.8 gene lead to peripheral neuropathic pain. Based on a series of animal experiments and human genetic evidence, selective inhibition of Nav1.8 holds potential as a novel analgesic therapy, applicable for treating various pain types including inflammatory pain, neuropathic pain, postoperative pain, and cancer pain.

A major drawback of some known Nav inhibitors is their poor therapeutic window, which may result from their lack of isoform selectivity. Since Nav1.8 is primarily restricted to pain-sensing neurons, selective Nav1.8 blockers are unlikely to induce the adverse effects commonly associated with non-selective Nav blockers. Therefore, there remains a need in the art to develop new Nav1.8 selective inhibitors, preferably Nav channel inhibitors with better selectivity for Nav1.8, greater efficacy, increased metabolic stability, increased solubility, and fewer side effects.

Chinese Patent Application CN202310743287.6 discloses the structure of a compound of formula I:

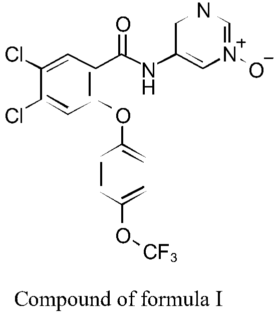

The compound of formula I can effectively antagonize Nav1.8 receptor activity and holds broad application prospects in the preparation of medicaments for treating diseases associated with Nav1.8. Therefore, further research on the compound of formula I and its salt forms and crystal forms is of significant importance for developing effective therapeutic drugs.

### SUMMARY

In order to solve the problems in the prior art, in one aspect, the present disclosure provides a crystal form of a compound of formula I or a pharmaceutically acceptable salt thereof, wherein the compound of formula I has a structure shown below:

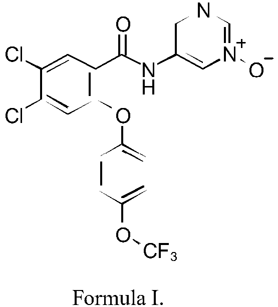

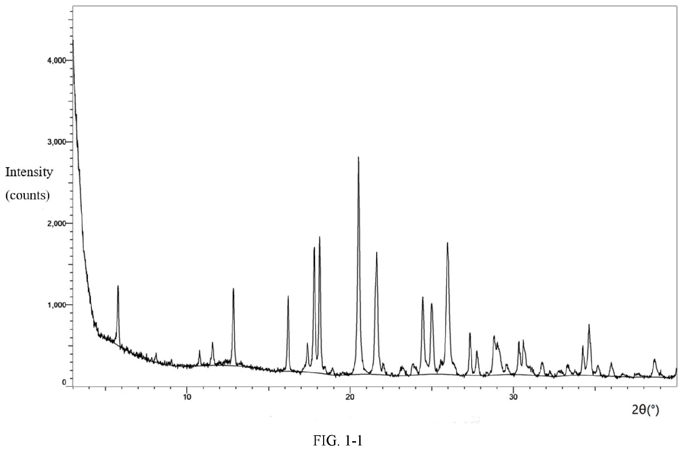

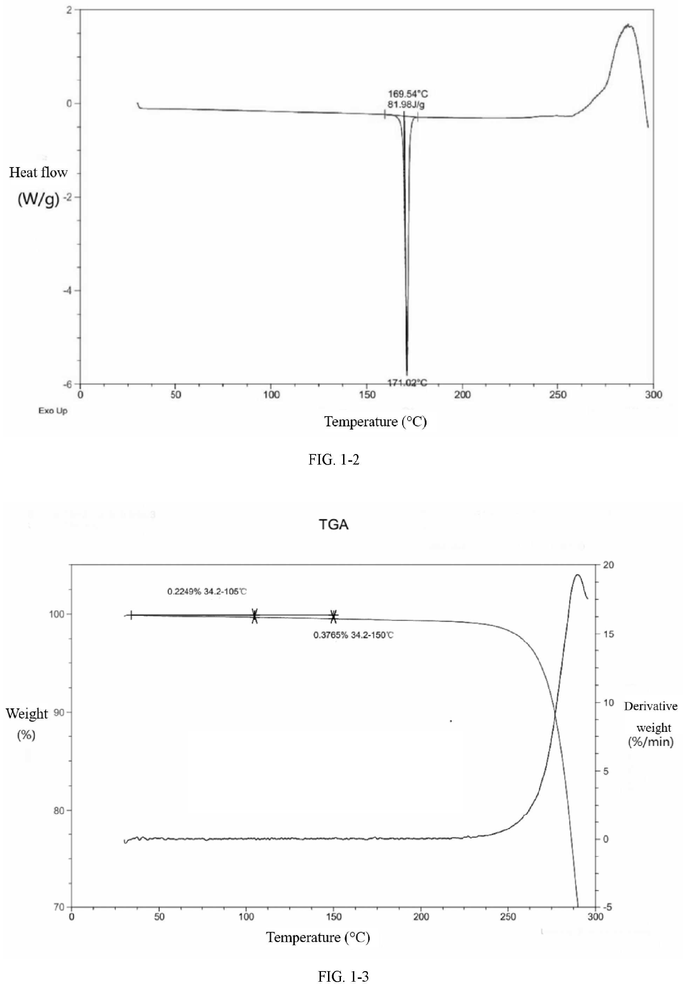

In some embodiments, the present disclosure provides the free crystal form A of the compound of formula I, and the free crystal form A has an X-ray powder diffraction pattern comprising diffraction peaks at 2θ±0.2° diffraction angles of 17.79°, 18.13°, 20.52°, 21.63°, and 25.97°; further, the free crystal form A has an X-ray powder diffraction pattern comprising diffraction peaks at 2θ±0.2° diffraction angles of one or more of the following: 12.85°, 16.20°, 24.46°, and 25.00°; further, the free crystal form A has an X-ray powder diffraction pattern comprising diffraction peaks at 2θ±0.2° diffraction angles of 12.85°, 16.20°, 17.79°, 18.13°, 20.52°, 21.63°, 24.46°, 25.00°, and 25.97°; furthermore, the free crystal form A has an X-ray powder diffraction pattern comprising diffraction peaks at 2θ±0.2° diffraction angles of 5.78°, 11.56°, 12.85°, 16.20°, 17.39°, 17.79°, 18.13°, 20.52°, 21.63°, 24.46°, 25.00°, 25.97°, 27.33°, 27.77°, 28.83°, 29.03°, 30.33°, 30.62°, 34.25°, and 34.64°; still further, the free crystal form A has an X-ray powder diffraction pattern comprising diffraction peaks at 2θ±0.2° diffraction angles of 5.78°, 10.77°, 11.56°, 12.85°, 16.20°, 17.39°, 17.79°, 18.13°, 20.52°, 21.63°, 22.01°, 24.46°, 25.00°, 25.56°, 25.97°, 27.33°, 27.77°, 28.83°, 29.03°, 30.33°, 30.62°, 31.77°, 33.33°, 34.25°, 34.64°, 35.19°, 36.01°, and 38.66°; yet further, the free crystal form A has an X-ray powder diffraction pattern comprising diffraction peaks at 2θ±0.2° diffraction angles of 5.78°, 8.10°, 9.06°, 10.77°, 11.56°, 12.85°, 16.20°, 17.39°, 17.79°, 18.13°, 18.45°, 18.93°, 19.55°, 20.52°, 21.63°, 22.01°, 23.20°, 23.89°, 24.46°, 25.00°, 25.56°, 25.97°, 26.39°, 27.33°, 27.77°, 28.83°, 29.03°, 29.60°, 30.33°, 30.62°, 31.14°, 31.77°, 32.23°, 33.33°, 33.77°, 34.25°, 34.64°, 35.19°, 36.01°, 38.66°, and 32.86°; yet further, the free crystal form A has an XRPD pattern substantially as shown in FIG. 1-1.

In some embodiments, the free crystal form A has one, two, or three of the following characteristics:
(1) a TGA curve of the free crystal form A showing a weight loss of about 0.38±1% at 150.0±3 °C;
(2) a DSC curve of the free crystal form A having a starting point of an endothermic peak at 169.5±3 °C; and
(3) a DSC curve of the free crystal form A having one endothermic peak at 171.0±3 °C.

In some embodiments, the DSC diagram of the free crystal form A is as shown in FIG. 1-2; the TGA diagram of the free crystal form A is as shown in FIG. 1-3.

According to an embodiment of the present disclosure, the free crystal form A is an anhydrous crystal form.

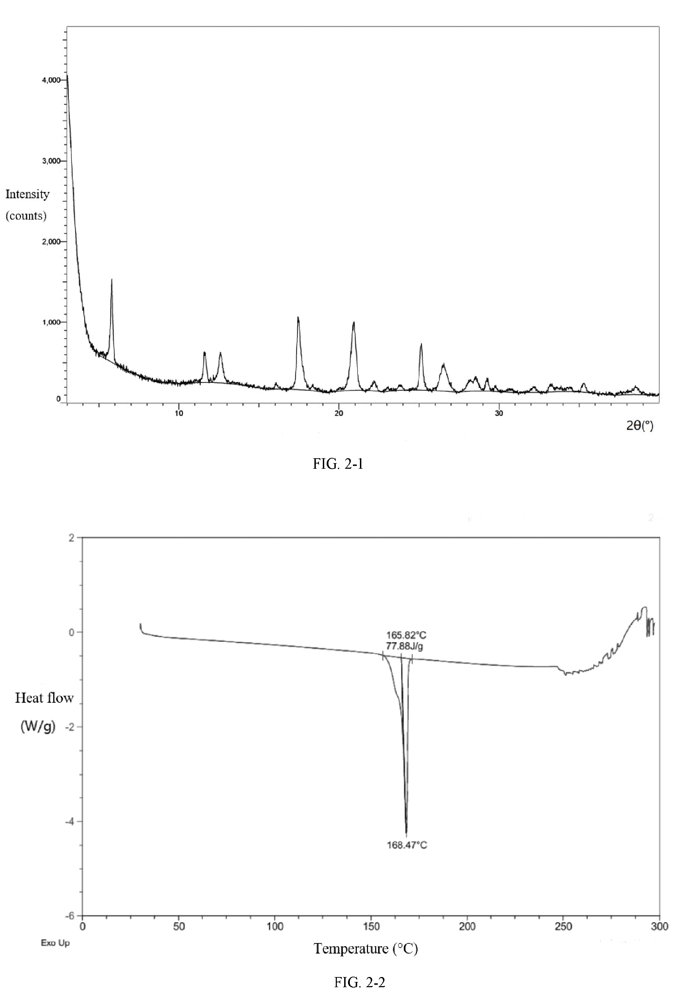

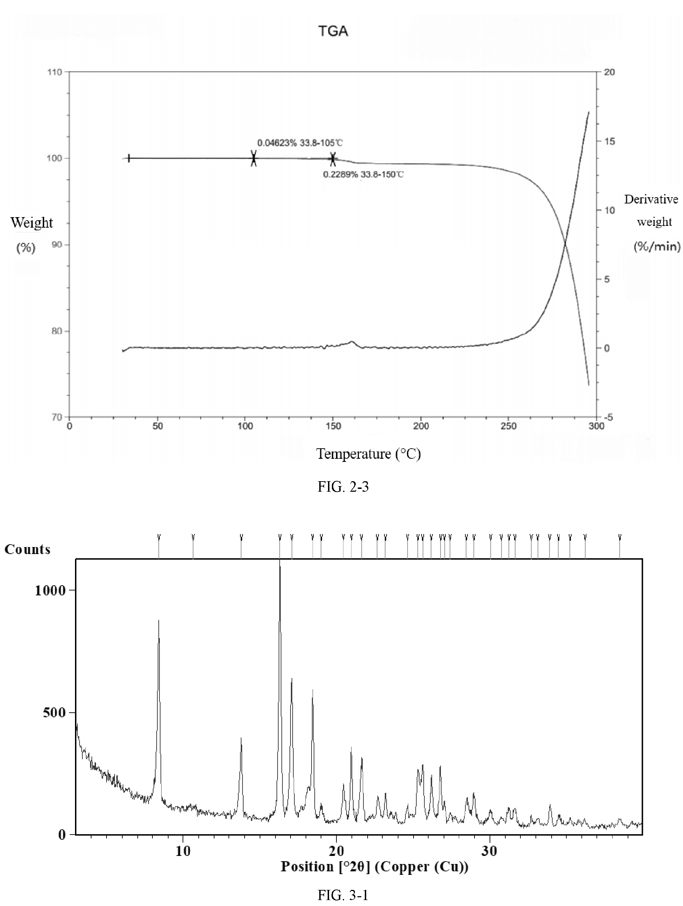

In some embodiments, the present disclosure provides the free crystal form B of the compound of formula I, and the free crystal form B has an X-ray powder diffraction pattern comprising diffraction peaks at 2θ±0.2° diffraction angles of 11.64°, 12.60°, 17.46°, 20.93°, 25.16°, and 26.56°; further, the free crystal form B has an X-ray powder diffraction pattern comprising diffraction peaks at 2θ±0.2° diffraction angles of one or more of 5.80°, 29.25°, 28.22°, 28.51°, and 35.32°; furthermore, the free crystal form B has an X-ray powder diffraction pattern comprising diffraction peaks at 2θ±0.2° diffraction angles of 5.80°, 11.64°, 12.60°, 17.46°, 20.93°, 22.19°, 25.16°, 26.56°, 28.22°, 28.51°, 29.25°, 33.23°, 35.32°, and 38.52°; still further, the free crystal form B has an X-ray powder diffraction pattern comprising diffraction peaks at 2θ±0.2° diffraction angles of 5.80°, 11.64°, 12.60°, 16.09°, 17.46°, 18.37°, 20.93°, 22.19°, 23.03°, 23.82°, 25.16°, 26.56°, 28.22°, 28.51°, 29.25°, 29.79°, 32.20°, 33.23°, 34.42°, 35.32°, and 38.52°; yet further, the free crystal form B has an XRPD pattern substantially as shown in FIG. 2-1.

In some embodiments, the free crystal form B has one, two, or three of the following characteristics:
(1) a TGA curve of the free crystal form B showing a weight loss of about 0.23±1% at 150.0±3 °C;
(2) a DSC curve of the free crystal form B having a starting point of an endothermic peak at 165.8±3 °C; and
(3) The DSC curve of the free crystal form B has an endothermic peak at 168.47±3°C.

In some embodiments, the DSC thermogram of the free crystal form B is as shown in FIG. 2-2; and the TGA thermogram of the free crystal form B is as shown in FIG. 2-3.

According to an embodiment of the present disclosure, the free crystal form B is an anhydrous crystal form.

In another aspect, the present disclosure provides a preparation method for the free crystal form A of the compound of formula I, which comprises the following methods:
Method 1: placing a first sample vial containing the compound of formula I in an open state into a second sample vial containing a solvent, sealing the second sample vial, and allowing it to stand at room temperature; wherein the solvent does not submerge the mouth of the first sample vial;
   wherein the solvent is selected from one or more of ethanol, acetone, methyl tert-butyl ether, ethyl acetate, dichloromethane, tetrahydrofuran, acetonitrile, n-heptane, and toluene;
Method 2: placing a first sample vial containing a solution of the compound of formula I in an open state in a second sample vial containing an anti-solvent, sealing the second sample vial, and allowing it to stand at room temperature; wherein the anti-solvent does not submerge the mouth of the first sample vial;
   wherein the solvent in the solution of the compound of formula I is selected from one or more of dichloromethane, tetrahydrofuran, 1,4-dioxane, dimethyl sulfoxide, and N,N-dimethylformamide;
   and the anti-solvent is selected from one or more of n-heptane, methyl tert-butyl ether, and water;
Method 3: adding a solvent to the compound of formula I at room temperature, stirring magnetically, and collecting the solid;
   wherein the solvent is selected from one or more of water, ethanol, isopropanol, ethyl acetate, isopropyl acetate, tetrahydrofuran, 1,4-dioxane, methyl tert-butyl ether, acetone, methyl ethyl ketone, dimethyl sulfoxide, acetonitrile, toluene, N,N-dimethylformamide, and N-methylpyrrolidone;
Method 4: adding a solvent to the compound of formula I at 50°C, stirring magnetically, and collecting the solid;
   wherein the solvent is selected from one or more of water, ethanol, isopropanol, ethyl acetate, isopropyl acetate, tetrahydrofuran, 1,4-dioxane, methyl tert-butyl ether, acetone, methyl ethyl ketone, dimethyl sulfoxide, acetonitrile, toluene, N,N-dimethylformamide, and N-methylpyrrolidone;
Method 5: adding the compound of formula I to an organic solvent I, dissolving, then filtering, and volatilizing at room temperature;
   preferably, the organic solvent I is selected from one or more of methanol, ethanol, ethyl acetate, tetrahydrofuran, 1,4-dioxane, acetone, methyl ethyl ketone, dichloromethane, and acetonitrile;
Method 6: completely dissolving the compound of formula I in a good solvent, filtering, and adding an anti-solvent dropwise to the clear solution until a solid precipitates;
   wherein the good solvent is selected from one or more of dimethyl sulfoxide, N,N-dimethylformamide, dichloromethane, tetrahydrofuran, methyl ethyl ketone, N-methylpyrrolidone, N,N-dimethylacetamide, ethanol, and isopropyl acetate;
   and the anti-solvent is selected from one or more of water, toluene, methyl tert-butyl ether, and n-heptane;
   In some embodiments, when the anti-solvent is selected from water, the good solvent is selected from one of dimethyl sulfoxide, N,N-dimethylformamide, tetrahydrofuran, N-methylpyrrolidone, and N,N-dimethylacetamide; when the anti-solvent is selected from methyl tert-butyl ether, the good solvent is selected from dichloromethane; and when the anti-solvent is selected from n-heptane, the good solvent is selected from methyl ethyl ketone or ethanol.
Method 7: completely dissolving the compound of formula I in a good solvent, filtering, and adding the clear solution to an anti-solvent;
   wherein the good solvent is selected from one or more of dimethyl sulfoxide, N,N-dimethylformamide, dichloromethane, ethyl acetate, 1,4-dioxane, methyl ethyl ketone, and tetrahydrofuran;
   and the anti-solvent is selected from one or more of water, n-heptane, methyl tert-butyl ether, and toluene;
   In some embodiments, when the anti-solvent is selected from water, the good solvent is selected from dimethyl sulfoxide or N,N-dimethylformamide; when the anti-solvent is selected from methyl tert-butyl ether, the good solvent is selected from 1,4-dioxane or methyl ethyl ketone; when the anti-solvent is selected from n-heptane, the good solvent is selected from ethyl acetate; and when the anti-solvent is selected from toluene, the good solvent is selected from methyl ethyl ketone or tetrahydrofuran.
Method 8: adding the compound of formula I to an organic solvent I at 50°C, dissolving, filtering, magnetically stirring, and cooling to room temperature;
   preferably, the organic solvent I is selected from one or more of methanol, ethanol, isopropyl acetate, acetonitrile, ethyl acetate, and acetone;
Method 9: adding the compound of formula I to a mortar, or adding the compound of formula I and a solvent to a mortar, and grinding;
   preferably, the solvent is selected from one or more of water, methyl tert-butyl ether, and n-heptane.

In another aspect, the present disclosure provides a preparation method for the free crystal form B of the compound of formula I, which comprises the following several methods:
Method 1: completely dissolving the compound of formula I in a good solvent, filtering, and dropwise adding an anti-solvent to the clear solution until a solid precipitates;
   wherein the good solvent is selected from one or two of tetrahydrofuran, N,N-dimethylacetamide, and the anti-solvent is selected from n-heptane;
   and the anti-solvent is selected from one or more of water, toluene, methyl tert-butyl ether, n-heptane.
Method 2: completely dissolving the compound of formula I in a good solvent, filtering, and adding the clear solution to an anti-solvent;
   wherein the good solvent is selected from one or two of dichloromethane, 1,4-dioxane; and the anti-solvent is selected from n-heptane.
Method 3: The compound of formula I is added to isopropanol at 40-60°C (e.g., 50°C), dissolved, filtered, stirred, and cooled to room temperature.
Method 4: completely dissolving the compound of formula I in tetrahydrofuran, filtering, adding n-heptane to the clear solution under stirring until a solid precipitates, adding seed crystals of the free crystal form B seeds, and stirring.

According to the embodiments of the present disclosure, the seed crystals of the free crystal form B can be prepared by one of the methods 1, 2, 3, and 4.

In another aspect, the present disclosure provides a pharmaceutically acceptable salt of the compound of formula I, wherein the pharmaceutically acceptable salt is a salt formed from the compound of formula I with an acid or a base; in some embodiments, the pharmaceutically acceptable salt of the compound of formula I is a salt formed from the compound of formula I with the following acid or base: hydrochloric acid, sulfuric acid, maleic acid, phosphoric acid, fumaric acid, tartaric acid, citric acid, L-malic acid, succinic acid, p-toluenesulfonic acid, methanesulfonic acid, sodium hydroxide, or arginine.

When the compound of formula I forms a salt with an acid or a base, the acid or base and the compound of formula I may be in a molar ratio of 5:1 to 1:5, e.g., 5:1, 4.5:1, 4:1, 3.5:1, 3:1, 2.5:1, 2:1, 1.5:1, 1:1, 1:1.5, 1:2, 1:2.5, 1:3, 1:3.5, 1:4, 1:4.5, or 1:5.

In some embodiments, the pharmaceutically acceptable salt of the compound of formula I is maleate of the compound of formula I; in some embodiments, in the maleate, the compound of formula I and maleic acid are in a molar ratio of 1:1.

In some embodiments, the pharmaceutically acceptable salt of the compound of formula I is sodium salt of the compound of formula I; in some embodiments, in the sodium salt, the compound of formula I and sodium are in a molar ratio of 1:1.

In another aspect, the present disclosure provides a crystal form of the pharmaceutically acceptable salt of the compound of formula I.

In some embodiments, the present disclosure provides crystal form A of the maleate of the compound of formula I, and the crystal form A of the maleate has an X-ray powder diffraction pattern comprising diffraction peaks at 2θ±0.2° diffraction angles of 8.39°, 13.78°, 16.31°, 17.07°, and 18.44°; further, the crystal form A of the maleate has an X-ray powder diffraction pattern comprising diffraction peaks at 2θ±0.2° diffraction angles of 8.39°, 13.78°, 16.31°, 17.07°, 18.44°, 20.96°, 25.62°, and 26.78°; further, the crystal form A of the maleate has an X-ray powder diffraction pattern comprising diffraction peaks at 2θ±0.2° diffraction angles of 8.39°, 13.78°, 16.31°, 17.07°, 18.44°, 20.96°, 21.61°, 25.29°, 25.62°, and 26.78°; further, the crystal form A of the maleate has an X-ray powder diffraction pattern comprising diffraction peaks at 2θ±0.2° diffraction angles of 8.39°, 13.78°, 16.31°, 17.07°, 18.44°, 20.45°, 20.96°, 21.61°, 25.29°, 25.62°, 26.19°, and 26.78°; further, the crystal form A of the maleate has an X-ray powder diffraction pattern comprising diffraction peaks at 2θ±0.2° diffraction angles of 8.39°, 13.78°, 16.31°, 17.07°, 18.44°, 19.01°, 20.45°, 20.96°, 21.61°, 22.65°, 23.19°, 24.62°, 25.29°, 25.62°, 26.19°, 26.78°, 27.03°, 27.41°, 28.47°, 28.96°, 30.05°, 31.25°, 31.61°, and 33.91°; further, the crystal form A of the maleate has an X-ray powder diffraction pattern comprising diffraction peaks at 2θ±0.2° diffraction angles of 8.39°, 10.65°, 13.78°, 16.31°, 17.07°, 18.44°, 19.01°, 20.45°, 20.96°, 21.61°, 22.65°, 23.19°, 24.62°, 25.29°, 25.62°, 26.19°, 26.78°, 27.03°, 27.41°, 28.47°, 28.96°, 30.05°, 30.74°, 31.25°, 31.61°, 32.70°, 33.13°, 33.91°, 34.49°, 35.22°, 36.21°, and 38.48°; further, the crystal form A of the maleate has an XRPD pattern substantially as shown in FIG. 3-1;

In some embodiments, the crystal form A of the maleate has one or two of the following characteristics:
(1) the TGA curve of the crystal form A of the maleate shows a weight loss of 0.5-3% at 120 ±3°C, preferably 0.5, 1.0, 1.5, 2.0, 2.5, or 3.0%, for example, 1.41%; and
(2) the crystal form A of the maleate has an endothermic peak at 139.1±3°C.

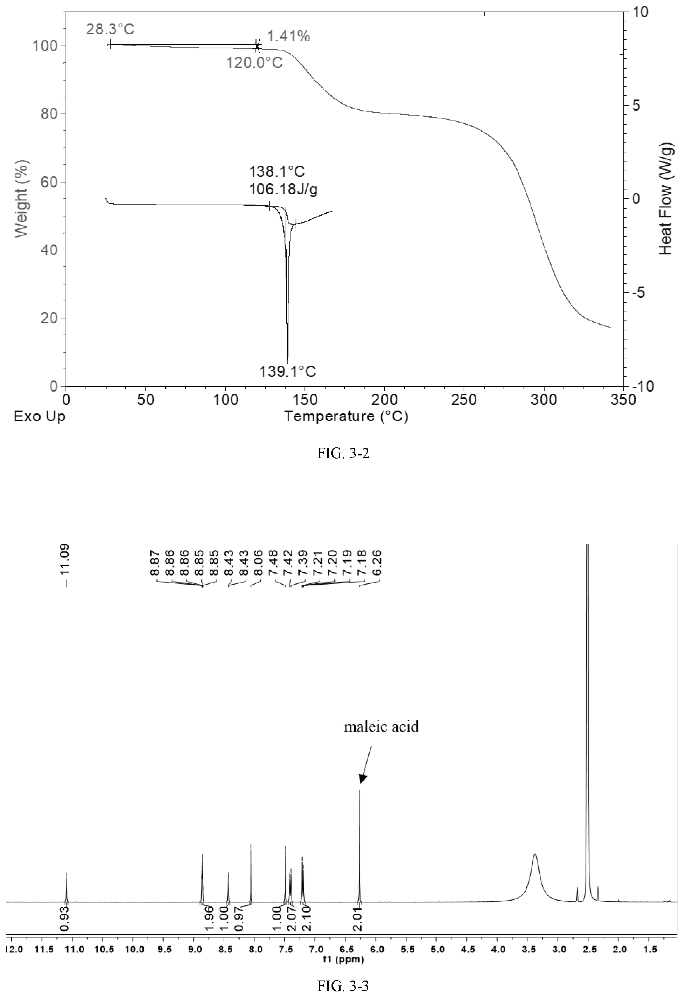

In some embodiments, a TGA/DSC profile of the crystal form A of the maleate is substantially as shown in FIG. 3-2; and a ¹H NMR spectrum of the crystal form A of the maleate is substantially as shown in FIG. 3-3.

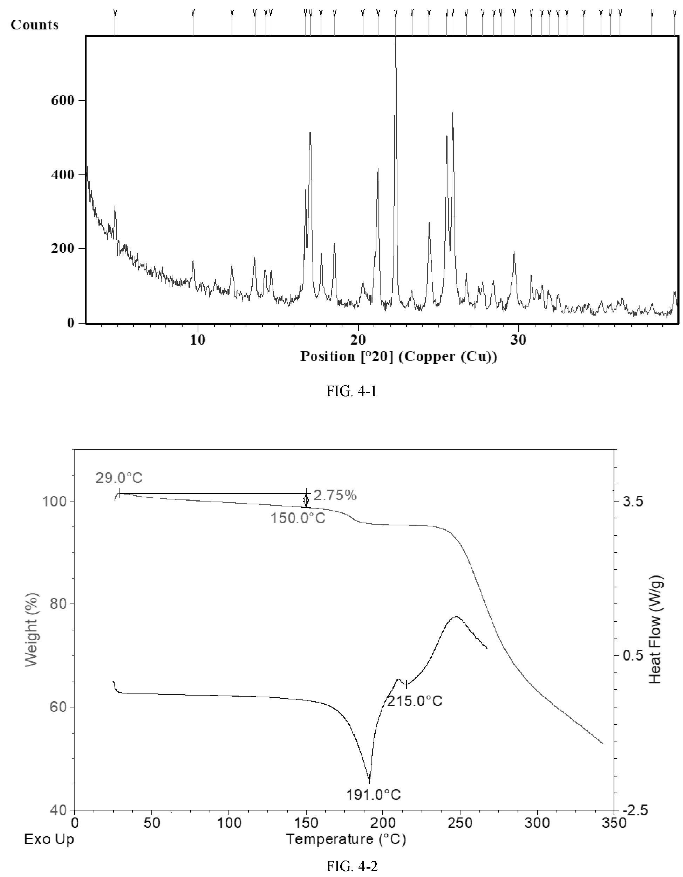

In some embodiments, the present disclosure provides a crystal form A of a sodium salt of a compound of formula I, and the crystal form A of the sodium salt has an X-ray powder diffraction pattern comprising diffraction peaks at 2θ±0.2° diffraction angles of 16.70°, 17.02°, 21.23°, 22.33°, 24.39°, 25.50°, and 25.89°; further, the crystal form A of the sodium salt has an X-ray powder diffraction pattern comprising diffraction peaks at 2θ±0.2° diffraction angles of 16.70°, 17.02°, 17.67°, 18.51°, 21.23°, 22.33°, 24.39°, 25.50°, 25.89°, and 29.73°; still further, the crystal form A of the sodium salt has an X-ray powder diffraction pattern comprising diffraction peaks at 2θ±0.2° diffraction angles of 16.70°, 17.02°, 17.67°, 18.51°, 21.23°, 22.33°, 24.39°, 25.50°, 25.89°, 29.73°, and 30.76°; yet further, the crystal form A of the sodium salt has an X-ray powder diffraction pattern comprising diffraction peaks at 2θ±0.2° diffraction angles of 4.85°, 9.70°, 12.13°, 13.55°, 14.20°, 14.55°, 16.70°, 17.02°, 17.67°, 18.51°, 21.23°, 22.33°, 24.39°, 25.50°, 25.89°, 26.72°, 27.74°, 28.44°, 29.73°, and 30.76°; yet further, the crystal form A of the sodium salt has an X-ray powder diffraction pattern comprising diffraction peaks at 2θ±0.2° diffraction angles of 4.85°, 9.70°, 12.13°, 13.55°, 14.20°, 14.55°, 16.70°, 17.02°, 17.67°, 18.51°, 20.27°, 21.23°, 22.33°, 23.35°, 24.39°, 25.50°, 25.89°, 26.72°, 27.74°, 28.44°, 29.73°, 30.76°, 31.43°, 31.88°, 32.46°, and 39.71°; yet further, the crystal form A of the sodium salt has an X-ray powder diffraction pattern comprising diffraction peaks at 4.85°, 9.70°, 12.13°, 13.55°, 14.20°, 14.55°, 16.70°, 17.02°, 17.67°, 18.51°, 20.27°, 21.23°, 22.33°, 23.35°, 24.39°, 25.50°, 25.89°, 26.72°, 27.74°, 28.44°, 28.87°, 29.73°, 30.76°, 31.43°, 31.88°, 32.46°, 32.98°, 34.06°, 35.13°, 35.68°, 36.33°, 38.30°, and 39.71°; and further, the crystal form A of the sodium salt has an XRPD pattern substantially as shown in FIG. 4-1.

In some embodiments, the crystal form A of the sodium salt has one or two of the following characteristics:
(1) a TGA curve of the crystal form A of the sodium salt shows a weight loss of 1.0-5.0% at 150 ±3°C, preferably 1.0, 1.5, 2.0, 2.5, 3.0, 3.5, 4.0, 4.5, or 5.0%, for example 2.75%; and
(2) the crystal form A of the sodium salt has two endothermic peaks at 191.0 ±3°C and 215.0 ±3°C.

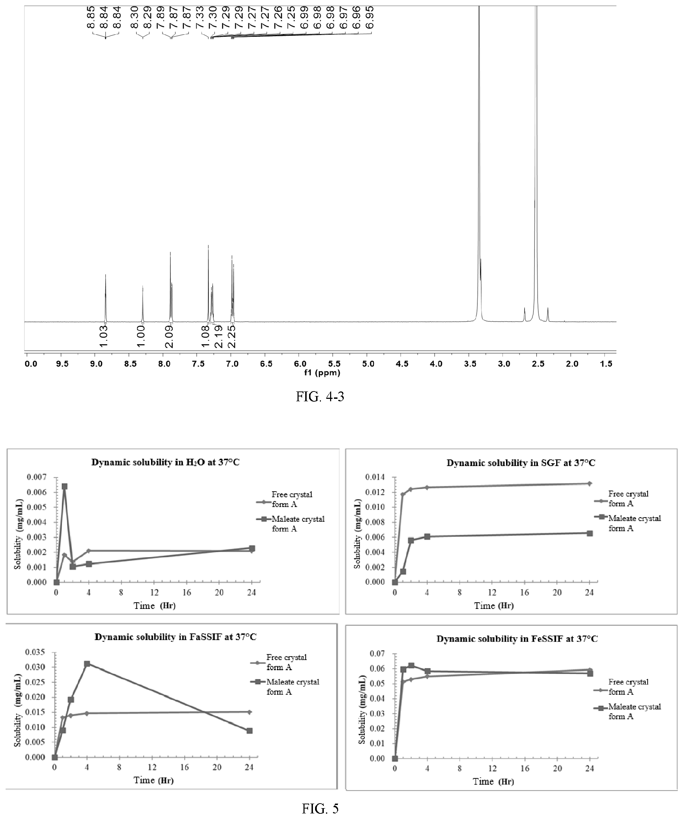

In some embodiments, a TGA/DSC profile of the crystal form A of the sodium salt is substantially as shown in FIG. 4-2; and a ¹H NMR spectrum of the crystal form A of the sodium salt is substantially as shown in FIG. 4-3.

In another aspect, the present disclosure provides a method for preparing the pharmaceutically acceptable salt (including the crystal form of the salt) of the compound of formula I, comprising mixing the compound of formula I with a suitable acid or base to obtain the pharmaceutically acceptable salt.

In some embodiments, when the pharmaceutically acceptable salt of the compound of formula I is a maleate or a sodium salt, the preparation method comprises the following steps: mixing the compound of formula I with an equimolar amount of maleic acid or sodium hydroxide in an organic solvent A under stirring, and isolating the resulting solid. In some embodiments, the stirring time is 1-5 days, for example, 3 days, and the stirring temperature is room temperature; the organic solvent A is selected from one or more of isopropanol, ethyl acetate, 2-methyltetrahydrofuran. In some embodiments, the compound of formula I is mixed with maleic acid or sodium hydroxide to obtain a suspension in the organic solvent A, which is then stirred as a suspension;

In some embodiments, when the pharmaceutically acceptable salt of the compound of formula I is maleate crystal form A, the preparation method comprises the following steps: mixing the compound of formula I with an equimolar amount of maleic acid to obtain a suspension in the organic solvent A, stirring the suspension, and isolating the resulting solid. In some embodiments, the suspension stirring time is 1-5 days, for example, 3 days, and the suspension stirring temperature is room temperature; the organic solvent A is ethyl acetate.

In some embodiments, when the pharmaceutically acceptable salt of the compound of formula I is sodium salt crystal form A, the preparation method comprises the following steps: mixing the compound of formula I with an equimolar amount of sodium hydroxide to obtain a suspension in the organic solvent A, stirring the suspension, and isolating the resulting solid. In some embodiments, the suspension stirring time is 1-5 days, for example, 3 days, and the suspension stirring temperature is room temperature; the organic solvent A is selected from one or more of isopropanol, ethyl acetate, 2-methyltetrahydrofuran.

In yet another aspect, the present disclosure provides a pharmaceutical composition comprising one or more of the free crystal forms (e.g., free crystal form A, free crystal form B) of the compound of formula I as described above and the pharmaceutically acceptable salt (including the crystal form thereof) of the compound of formula I as described above.

In some embodiments, the pharmaceutical composition further comprises a pharmaceutically acceptable excipient or carrier.

In another aspect, the present disclosure provides a pharmaceutical composition comprising substance A and a pharmaceutically acceptable carrier, wherein the substance A is a free crystal form of the compound of formula I or a crystal form of a pharmaceutically acceptable salt of the compound of formula I as described in any one of the preceding items; preferably, the substance A is free crystal form A of the compound of formula I, free crystal form B of the compound of formula I, maleate crystal form A, or sodium salt crystal form A.

In another aspect, the present disclosure provides a use of the free crystal form of the compound of formula I (e.g., free crystal form A, free crystal form B), a pharmaceutically acceptable salt of the compound of formula I (including its crystalline forms), or the pharmaceutical composition in the manufacture of a medicament for treating and/or preventing a voltage-gated sodium ion channel-related disease.

According to an embodiment of the present disclosure, the voltage-gated sodium ion channel-related disease is a Nav1.8-related disease.

According to an embodiment of the present disclosure, the use of the free crystal form of the compound of formula I (e.g., free crystal form A, free crystal form B), a pharmaceutically acceptable salt of the compound of formula I (including its crystalline forms), or the pharmaceutical composition of the present disclosure can provide patients in need with a superior and more effective clinical treatment drug or regimen.

The present disclosure also provides a method for treating and/or preventing a Nav1.8-related disease, the method comprising administering to a patient a therapeutically effective amount of a crystalline form of the compound of formula I as described above, a pharmaceutically acceptable salt of the compound of formula I as described above, a crystalline form of the salt as described above, or the pharmaceutical composition as described above; preferably, comprising a pharmaceutical formulation of the free crystal form of the compound of formula I as described above (e.g., free crystal form A, free crystal form B), a pharmaceutically acceptable salt of the compound of formula I (including its crystalline forms, e.g., maleate crystal form A, sodium salt crystal form A), or the pharmaceutical composition as described above.

According to an embodiment of the present disclosure, the Nav1.8-related diseases include: pain.
preferably, the pain comprises: acute pain, chronic pain, inflammatory pain, cancer pain, neuropathic pain, musculoskeletal pain, primary pain, intestinal pain, or idiopathic pain.

### Definitions and Explanations of Terms

The various terms and phrases used in the present disclosure have the general meanings known to those skilled in the art. Nevertheless, the present disclosure still intends to provide a more detailed explanation and interpretation of these terms and phrases herein. If the meaning of any mentioned term or phrase is inconsistent with the commonly known meaning, the meaning expressed in the present disclosure shall prevail.

Unless otherwise stated, a numerical range set forth in the specification and claims shall be construed as at least including each specific integer value within the range. For example, "two or more" represents 2, 3, 4, 5, 6, 7, 8, 9, 10 or more. For example, when certain numerical ranges are defined or understood as "numbers", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

When "about" or "approximately" a certain numerical value is recited in the specification and claims, it shall be construed as including the numerical value itself, as well as numerical values within a range around the numerical value that is acceptable in the art, for example, numerical values within the range of ±15% of the numerical value, numerical values within the range of ±10% of the numerical value, numerical values within the range of ±5% of the numerical value, and the like. For example, about 10 represents that it includes a numerical value within the range of 10±1.5, i.e., within the range of 8.5 to 11.5; a numerical value within the range of 10±1.0, i.e., within the range of 9.0 to 11.0; and a numerical value within the range of 10±0.5, i.e., within the range of 9.5 to 10.5.

The "2θ or 2θ angle" described in the present disclosure refers to the diffraction angle, where θ is the Bragg angle, and the unit is ° or degree; the error range for each characteristic peak 2θ is ±0.20 (preferably ±0.10) (including cases where numbers exceeding two decimal places have been rounded).

The salt or polymorph of the compound of formula I of the present disclosure can be used in combination with other active ingredients, provided that it does not cause other adverse effects, such as allergic reactions.

The term "composition" as used in the present disclosure means a product comprising a specified amount of each specified ingredient, and any product that results, directly or indirectly, from the combination of the specified amounts of the specified ingredients.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition (i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms).

The term "pharmaceutically acceptable" means that a formula component or an active ingredient does not unduly and adversely affect a general therapeutic target's health.

The term "pharmaceutically acceptable excipient or carrier" means one or more compatible solid or liquid filler or gel substances that are suitable for human use and must be of sufficient purity and sufficiently low toxicity. "Compatible" herein means that the components in the composition can be blended with the compound of the present disclosure and with each other without substantially reducing the efficacy of the compound. Examples of pharmaceutically acceptable excipients or carriers include, but are not limited to, cellulose and derivatives thereof (such as sodium carboxymethylcellulose, sodium ethylcellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers, wetting agents (such as sodium lauryl sulfate), coloring agents, flavoring agents, stabilizers, antioxidants, preservatives, pyrogen-free water, and the like. The pharmaceutical composition can be specially formulated for oral administration in solid or liquid form, for parenteral injection, or for rectal administration. The pharmaceutical composition can be formulated into various dosage forms for convenient administration, for example, oral preparations (such as tablets, capsules, solutions or suspensions), injectable preparations (such as injectable solutions or suspensions, or injectable dry powders that can be used immediately after adding a pharmaceutical vehicle before injection).

For the above therapeutic and/or prophylactic uses, the total daily dosage of the salt, polymorph, and pharmaceutical composition of the compound of formula I of the present disclosure must be determined by the attending physician within the scope of sound medical judgment. For any particular patient, the specific therapeutically effective dosage level will depend upon a variety of factors, including the disorder being treated and the severity of the disorder; the activity of the specific compound employed; the specific composition employed; the age, body weight, general health, sex, and diet of the patient; the time of administration, route of administration, and rate of excretion of the specific compound employed; the duration of the treatment; drugs used in combination or concurrently with the specific compound employed; and like factors well known in the medical arts. For example, it is common practice in the art to start with a dose of a compound below the level required to achieve the desired therapeutic effect and gradually increase the dose until the desired effect is obtained.

The term "free form" refers to the form of a compound that is not further converted into a salt, such as the compound of formula I itself as described herein. Those skilled in the art will understand that when a "free form" forms a salt with a certain acid, the "free form" can be understood as the "free base" corresponding to the salt formed by that acid. That is, "free base" and "free form" have equivalent meanings; similarly, "free acid" can also have equivalent meaning to "free form" (when the free form forms a salt with a base).

### Beneficial Effects

The present disclosure provides a salt of a compound of formula I, and a crystal form of the compound of formula I and a salt thereof, which have good pharmaceutical properties.

### BRIEF DESCRIPTION OF THE DRAWINGS

FIG. 1-1 is an XRPD pattern of the free crystal form A of the compound of formula I;
FIG. 1-2 is a DSC profile of the free crystal form A of the compound of formula I;
FIG. 1-3 is a TGA profile of the free crystal form A of the compound of formula I;
FIG. 2-1 is an XRPD pattern of the free crystal form B of the compound of formula I;
FIG. 2-2 is a DSC profile of the free crystal form B of the compound of formula I;
FIG. 2-3 is a TGA profile of the free crystal form B of the compound of formula I;
FIG. 3-1 is an XRPD pattern of the crystal form A of the maleate
FIG. 3-2 is a TGA/DSC profile of the crystal form A of the maleate
FIG. 3-3 is a ¹H NMR spectrum of the crystal form A of the maleate
FIG. 4-1 is an XRPD pattern of the crystal form A of the sodium salt
FIG. 4-2 is a TGA/DSC profile of the crystal form A of the sodium salt
FIG. 4-3 is a ¹H NMR spectrum of the crystal form A of the sodium salt
FIG. 5 is a dynamic solubility curve graph at 37 °C

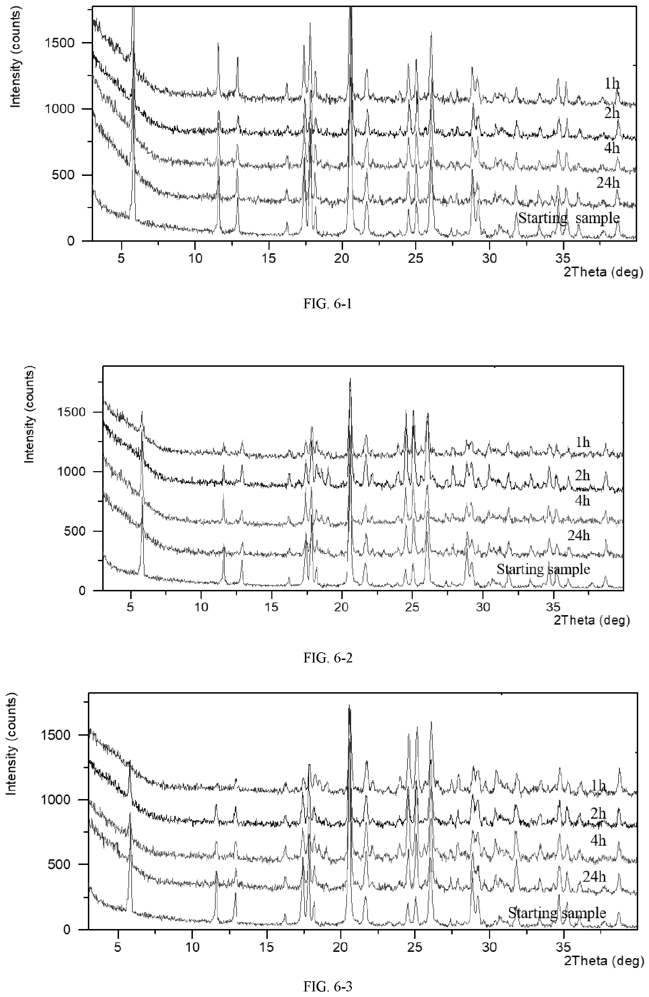

FIG. 6-1 is an XRPD overlay of the solubility samples of the free crystal form A in H₂O
FIG. 6-2 is an XRPD overlay of the solubility samples of the free crystal form A in SGF
FIG. 6-3 is an XRPD overlay of the solubility samples of the free crystal form A in FaSSIF

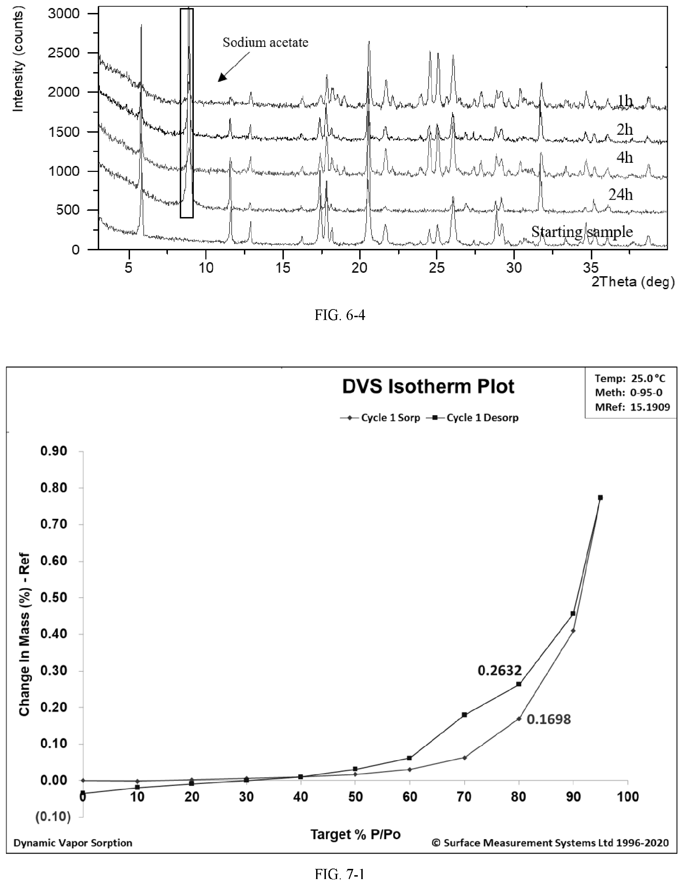

FIG. 6-4 is an XRPD overlay of the solubility samples of the free crystal form A in FeSSIF
FIG. 7-1 is a DVS profile of the free crystal form A

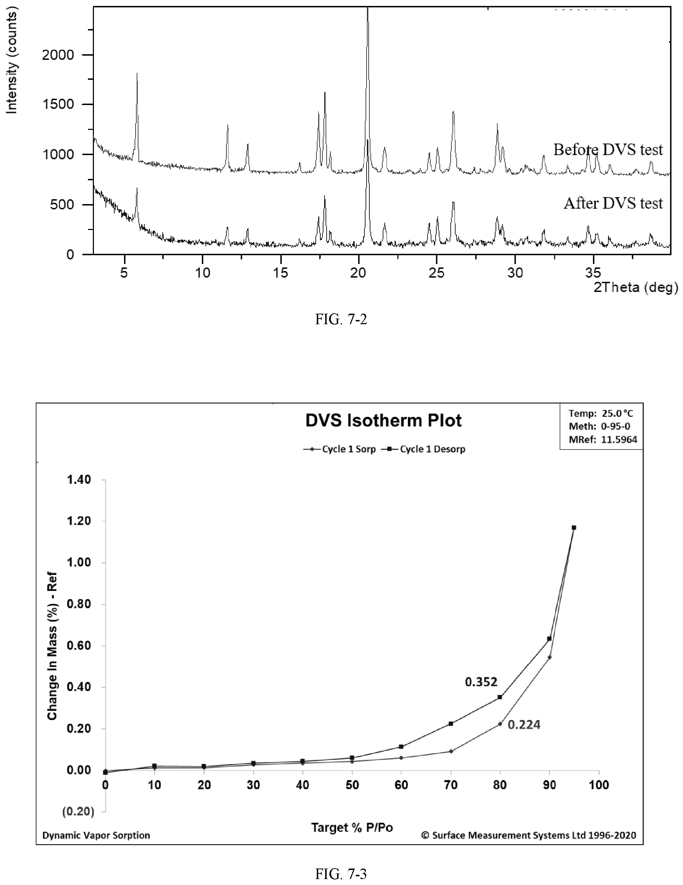

FIG. 7-2 is an XRPD overlay of the free crystal form A before and after the DVS test
FIG. 7-3 is a DVS profile of the crystal form A of the maleate

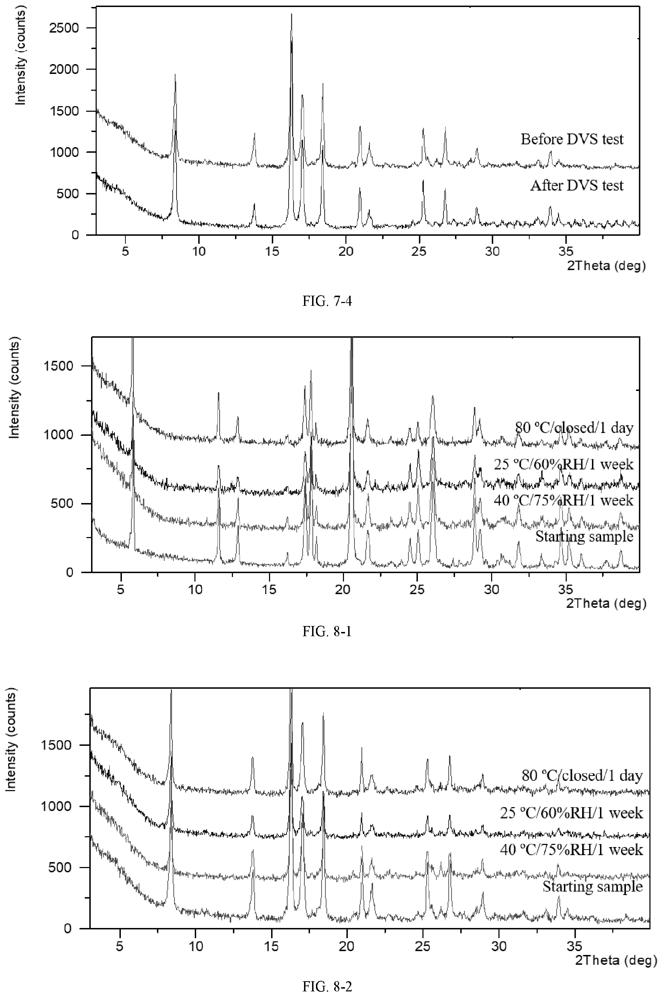

FIG. 7-4 is an XRPD overlay of the crystal form A of the maleate before and after the DVS test.
FIG. 8-1 is an XRPD overlay of samples of the free crystal form A for stability evaluation.
FIG. 8-2 is an XRPD overlay of samples of the crystal form A of the maleate for stability evaluation.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods.

### 1. Instruments and Detection Methods Adopted in the Present Disclosure as Below:

### 1. X-ray powder diffraction (XRPD)

XRPD patterns were acquired on X-ray powder diffraction analyzers manufactured by Bruker and PANalytacal, and the scanning parameters are shown in Table A-1a and Table A-1b below, respectively.

**Table A-1a. XRPD test parameters**

| Bruker model | **D8 ADVANCE** |
|---|---|
| X-ray | Cu, Kα, Kα1 (Å): 1.54060, Kα2 (Å): 1.54439 |
| | Kα2/Kα1 intensity ratio: 0.50 |
| X-ray light tube settings | 40 kV, 40 mA |
| Divergence slit | 0.300° |
| Scanning mode | Continuous |
| Scanning range (°2Theta) | 3-40 |
| Scanning time per step (s) | 32.00 |
| Scanning step size (°2Theta) | 0.019 |
| Test time | ~6 min |

**Table A-1b. XRPD test parameters**

| **Parameter** | **XRPD** |
|---|---|
| PANalytacal model | Empyrean/X' Pert3 |
| X-ray | Cu, Ka, |
| | Kα1 (Å): 1.540598, |
| | Kα2 (Å): 1.544426 |
| | Kα2/Kα1 intensity ratio: 0.50 |
| X-ray light tube settings | 45 kV, 40 mA |
| Divergence slit | 1/8° |
| Scanning mode | Continuous |
| Scanning range (°2θ) | 3-40 |
| Scanning time per step (s) | 46.7 |
| Scanning step length (°2θ) | 0.0263 |
| Test time | ~5 min |

### II. Thermogravimetric Analysis (TGA) and Differential Scanning Calorimetry (DSC)

2.1 TGA and DSC profiles were acquired on a TA Q500 thermogravimetric analyzer and a TA Q2000 differential scanning calorimeter, respectively, and the test parameters are listed in Table A-2a below.

**Table A-2a DSC and TGA Test Parameters**

| **Parameter** | **TGA** | **DSC** |
|---|---|---|
| Method | Linear heating | Linear heating |
| Sample pan | Aluminum pan, uncovered | Aluminum pan, crimped |
| Temperature range | 30 °C -setting endpoint temperature | 30 °C-setting endpoint temperature |
| Purging rate (°C/min) | 10 | 10 |
| Protective gas | Nitrogen | Nitrogen |

2.2 TGA and DSC profiles were acquired on a TA 5500 thermogravimetric analyzer and a TA 2500 differential scanning calorimeter, respectively, and the test parameters are listed in Table A-2b below.

**Table A-2b TGA and DSC Test Parameters**

| **Parameter** | **TGA** | **DSC** |
|---|---|---|
| Method | Linear heating | Linear heating |
| Sample pan | Aluminum pan, uncovered | Aluminum pan, crimped / uncrimped |
| Temperature range | Room temperature-setting endpoint temperature | 25 °C-setting endpoint temperature |
| Purging rate (°C/min) | 10 | 10 |
| Protective gas | Nitrogen | Nitrogen |

### III. Dynamic Vapor Sorption (DVS)

Dynamic vapor sorption (DVS) curves were acquired on a DVS IntrInsic from Surface Measurement Systems (SMS). The relative humidity at 25 °C was corrected with the deliquescence points of LiCl, Mg(NO3)2, and KCl. The DVS test parameters are listed in Table A-3.

**Table A-3. DVS test parameters**

| **Parameter** | **Set value** |
|---|---|
| Temperature | 25 °C |
| Sample amount | 10-20 mg |
| Protective gas and flow rate | N₂, 200 mL/min |
| dm/dt | 0.002%/min |
| Minimum dm/dt equilibration time | 10 min |
| Maximum equilibration time | 180 min |
| RH range | 0%RH-95%RH |
| RH gradient | 10%RH (0%RH ~ 90%RH & 90%RH ~ 0%RH) |
| | 5%RH (90%RH ~ 95%RH & 95%RH ~ 90%RH) |

### IV. Liquid-State Nuclear Magnetic Resonance (¹H NMR)

The liquid-state nuclear magnetic resonance spectra were acquired on a Bruker 400M nuclear magnetic resonance spectrometer with DMSO-d6 as a solvent.

### 5. Ultra performance liquid chromatography and ion chromatography (UPLC/IC)

In the study, the purity test, dynamic solubility test, and stability test were performed using a Waters H-Class ultra performance liquid chromatography system, and the ionic salt molar ratio test was performed using ion chromatography. The analytical conditions are shown in Table A-4 and Table A-5.

**Table A-4. High performance liquid chromatography test conditions**

| | | |
|---|---|---|
| **Liquid chromatography system** | **Waters H-Class chromatography system** | |
| Chromatographic column | Acquity UPLC CSH C18 2.1 mm/50 mm/1.7 um | |
| Mobile phase | A: 0.1% TFA in H₂O | |
| | B: 0.1% TFA in ACN | |
| | Time (min) | %B |
| | 0.0 | 10 |

| **Liquid** chromatography system | **Waters** H-Class chromatography system | |
|---|---|---|
| | 3.0 | 40 |
| | 9.0 | 50 |
| | 10.0 | 95 |
| | 11.0 | 95 |
| | 11.1 | 10 |
| | 13.0 | 10 |
| Run time | 13 min | |
| Flow rate of mobile phase | 0.5 mL/min | |
| Injection volume | 2 µL | |
| Detection wavelength | UV at 248 nm | |
| Column temperature | 30 °C | |
| Injector temperature | RT | |
| Diluent | ACN: H₂O=1/1 (v/v) | |

**Table A-5. Ion chromatography test conditions**

| **Ion chromatography system** | **Thermo AQ RFIC** |
|---|---|
| Chromatographic column | Dionex IonPac^{™} CS12A RFIC^{™} 4×250mm Analytical |
| Mobile phase | 20 mM Methanesulfonic acid |
| Injection volume | 25 µL |
| Flow rate | 1.0 mL/min |
| Temperature | 35 °C |
| Column temperature | 35 °C |
| Current | 80 mA |
| Run time | 7 min |

### 6. Preparation of Biological Media

### 3.1. Preparation of simulated gastric fluid (SGF)

100 mg of NaCl and 50 mg of Triton X-100 were weighed into a 50 mL volumetric flask and then completely dissolved by addition of purified water. 816 µL of 1 M hydrochloric acid was added, and the pH was adjusted to 1.8 with 1 M hydrochloric acid or 1 M NaOH solution. The mixed solution was brought to the volume by addition of purified water.

### 3.2. Preparation of fasted state simulated intestinal fluid (FaSSIF)

340 mg of anhydrous NaH₂PO₄ and 620 mg of NaCl were weighed and added into a 100 mL volumetric flask, and then completely dissolved by addition of purified water. 55.44 µL of a 50% NaOH solution was then added, and the pH was adjusted to 6.5 with 1 M hydrochloric acid or 1 M NaOH solution. The mixed solution was brought to the volume by addition of purified water. Subsequently, 110 mg of SIF powder was weighed and added into a 50 mL volumetric flask and completely dissolved in the solution described above, and the mixed solution was brought to the volume.

### 3.3. Preparation of fed state simulated intestinal fluid (FeSSIF)

0.82 mL of glacial acetic acid and 1.18 g of NaCl were added to a 100 mL volumetric flask and completely dissolved by addition of purified water. 528 µL of a 50% NaOH solution was then added, the pH was adjusted to 5.0 with 1 M hydrochloric acid or 1 M NaOH solution, and the mixed solution was brought to the volume by addition of purified water. Subsequently, 560 mg of SIF powder was weighed into a 50 mL volumetric flask and completely dissolved in the solution described above, and the mixed solution was brought to the volume.

### 7. Reagents Used in the Present Disclosure as Shown in Table A-6 Below

**Table A-6. Names for abbreviations of solvents used in the tests**

| **Abbreviation** | **Name** | **Abbreviation** | **Name** |
|---|---|---|---|
| MeOH | Methanol | ACN | Acetonitrile |
| EtOH | Ethanol | DCM | Dichloromethane |
| IPA | Isopropanol | Ethyl formate | Ethyl formate |
| Acetone | Acetone | n-Heptane | n-Heptane |
| MIBK | Methyl isobutyl ketone | H₂O | Water |
| EtOAc | Ethyl acetate | DMSO | Anisole |
| IPAc | Isopropyl acetate | THF | Tetrahydrofuran |
| MTBE | Methyl tert-butyl ether | 2-MeTHF | 2-Methyltetrahydrofuran |
| 2,2,2-Trifluoroethanol | Trifluoroethanol | 1,2-Dimethoxyethane | Ethylene glycol dimethyl ether |
| Toluene | Toluene | 1,4-Dioxane | 1,4-Dioxane |
| Cyclohexane | Cyclohexane | Isopropyl ether | Isopropyl ether |
| NMP | N-Methylpyrrolidone | MeOAc | Methyl acetate |
| CHCl₃ | Trichloromethane | MEK | Butanone |
| 2-Hexanone | 2-Hexanone | Anisole | Anisole |
| Xylene | Xylene | Cumene | Cumene |
| n-Pentane | n-Pentane | t-BuOH | tert-Butanol |
| n-PrOH | n-Propanol | DMF | N,N-Dimethylformamide |
| DMAc | N,N-Dimethylacetamide | EA | Ethyl acetate |
| PE | Petroleum ether | m-CPBA | m-Chloroperoxybenzoic acid |
| HATU | 2-(7-Azabenzotriazol-1-yl)-N,N,N',N'-tetramethyluronium hexafluorophosphate | DIPEA | N,N-Diisopropylethylamine |

### Example 1: Preparation of Compound of Formula I

### 5-(4,5-Dichloro-2-(4-(trifluoromethoxy)phenoxy)benzamido)pyrimidine 1-oxide

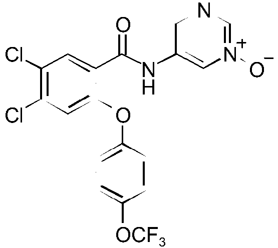

The synthetic route for the compound of Formula I is shown below:

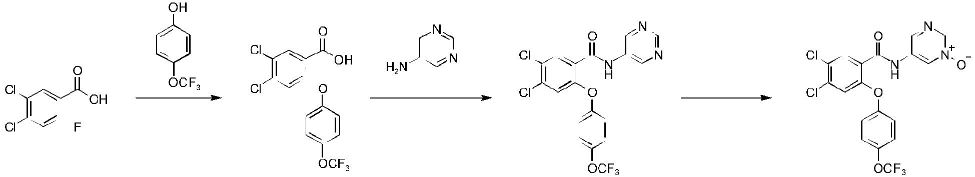

### Step 1: Synthesis of 4,5-dichloro-2-(4-(trifluoromethoxy)phenoxy)benzoic acid

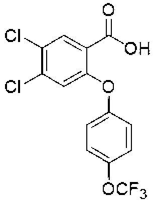

At room temperature, 4,5-dichloro-2-fluorobenzoic acid (1.0 g, 4.78 mmol), cesium carbonate (4.68 g, 14.35 mmol), and 4-(trifluoromethoxy)phenol (8 mL) were added to a 20 mL microwave tube. The tube was sealed and the reaction was carried out at 150°C for 1 hour. After cooling to room temperature, water (10 mL) was added to the reaction mixture, which was then extracted with EtOAc (20 mL × 3). The organic phase was washed once with saturated brine (20 mL), dried over anhydrous sodium sulfate, filtered, and concentrated under reduced pressure to dryness. The crude product was purified by column chromatography (SiO₂; PE:EA = 10: 1) to afford 4,5-dichloro-2-(4-(trifluoromethoxy)phenoxy)benzoic acid (3) (1.0 g; yield 56.9 %).

### Step 2: Synthesis of 4,5-dichloro-N-(pyrimidin-5-yl)-2-(4-(trifluoromethoxy)phenoxy)benzamide

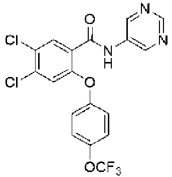

4,5-Dichloro-2-(4-(trifluoromethoxy)phenoxy)benzoic acid (1.0 g, 2.72 mmol), 5-aminopyrimidine (310.88 mg, 3.27 mmol), and HATU (2.07 g, 5.45 mmol) were added to DMF (10 mL). Then, DIPEA (1.06 g, 8.17 mmol) was added. After the addition was complete, the reaction mixture was stirred at room temperature for 16 hours. LC-MS indicated completion of the reaction. An NH₄Cl solution (15 mL) was added to the reaction mixture, which was then extracted with EtOAc (20 mL × 3). The combined organic phases were washed with water (20 mL × 2) and saturated brine (10 mL), dried over anhydrous sodium sulfate, filtered, and the filtrate was concentrated to dryness. The crude product was purified by normal-phase silica gel column chromatography (SiO₂; EtOAc/PE = 1:1) to afford 4,5-dichloro-N-(pyrimidin-5-yl)-2-(4-(trifluoromethoxy)phenoxy)benzamide (1.10 g; yield 90.9%).

### Step 3: Synthesis of 5-(4,5-dichloro-2-(4-(trifluoromethoxy)phenoxy)benzamido)pyrimidine 1-oxide (Compound of Formula I)

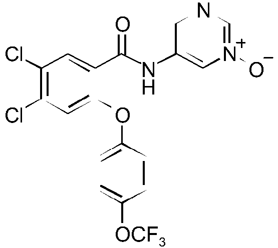

At room temperature, 4,5-dichloro-N-(pyrimidin-5-yl)-2-(4-(trifluoromethoxy)phenoxy)benzamide (1.0 g, 2.09 mmol) was weighed and dissolved in DCM (10 mL), followed by the slow addition of m-CPBA (849.89 mg, 4.19 mmol; 85%). The reaction mixture was stirred at room temperature for 16 hours. After completion of the reaction, the mixture was diluted with dichloromethane (20 mL) and washed with a saturated aqueous sodium bicarbonate solution (15 mL × 2). The organic phase was dried over Na₂SO₄, concentrated, and then subjected to preparative high-performance liquid chromatography (ammonia-acetonitrile) to afford 5-(4,5-dichloro-2-(4-(trifluoromethoxy)phenoxy)benzamido)pyrimidine 1-oxide (86.6 mg, yield 8.38 %).

¹H NMR (400 MHz, DMSO): δ 8.85-8.84 (m, 2H), 8.43-8.42 (m, 1H), 8.04 (s, 1H), 7.45 (s, 1H), 7.40-7.37 (m, 2H), 7.20-7.18 (m, 2H).

LC-MS, M/Z (ESI): 458.1 [M-H]⁻.

### Example 2: Preparation of a Crystal Form by Vapor-Solid Diffusion

Approximately 40 mg of the compound of formula **I** was weighed and placed into a 2 mL transparent sample vial, respectively. Separately, the solvent systems listed in Table 1-1 (3 mL each) were added to 20 mL sample vials. The open 2 mL transparent sample vials were placed inside the 20 mL sample vials, and the 20 mL sample vials were sealed and left to stand at room temperature. Solid samples were obtained by filtration. After vacuum drying at room temperature, the samples were subjected to XRPD testing. The results indicated that free crystal form A was obtained in all solvent systems listed in Table 1-1.

**Table 1-1: Crystal Form Screening Results by Vapor-Solid Diffusion**

| **Solvent System** | **Crystal form** |
|---|---|
| EtOH | Free Crystal Form A |
| Acetone | Free Crystal Form A |
| MTBE | Free Crystal Form A |
| EA | Free Crystal Form A |
| DCM | Free Crystal Form A |
| THF | Free Crystal Form A |
| ACN | Free Crystal Form A |
| Heptane | Free Crystal Form A |
| Toluene | Free Crystal Form A |

### Example 3: Preparation of a Crystal Form by Vapor-Liquid Diffusion

Approximately 40 mg of the compound of formula **I** was weighed and placed into a 2 mL transparent sample vial, respectively. A small amount of the good solvent listed in Table 2 was added to each 2 mL sample vial at room temperature, and the mixture was shaken to fully dissolve the sample. The resulting solution was filtered through a 0.22 µm filter membrane into another 2 mL transparent sample vial. Separately, a 20 mL sample vial was taken, and 4 mL of the corresponding anti-solvent listed in Table 2 was added to it. The open 2 mL transparent sample vial containing the filtered clear solution was placed inside this 20 mL sample vial. The 20 mL sample vial was sealed and left to stand at room temperature to precipitate solids. After vacuum drying at room temperature, the samples were subjected to XRPD testing. The results indicated that free crystal form A was obtained in all solvent systems listed in Table 1-2. When the good solvent was DMSO and the anti-solvent was H₂O, a mixture of free crystal forms A and B was obtained.

**Table 1-2: Crystal Form Screening Results by Vapor-Liquid Diffusion**

| **Solvent System** | | **Crystal form** |
|---|---|---|
| Good Solvent | Anti-solvent | |
| DCM | Heptane | Free Crystal Form A |
| DCM | MTBE | Free Crystal Form A |
| THF | Heptane | Free Crystal Form A |
| THF | MTBE | Free Crystal Form A |
| 1,4-Dioxane | Heptane | Free Crystal Form A |
| DMSO | H₂O | Free Crystal Forms A + B |
| DMF | H₂O | Free Crystal Form A |

### Example 4: Preparation of a Crystal Form by Room Temperature Slurry Method

Approximately 50 mg of the compound of formula **I** was weighed and placed into a 2 mL transparent sample vial, respectively. 0.1-0.5 mL of the solvent listed in Table 3 was added to each sample vial at room temperature, and the mixture was magnetically stirred for about two weeks. Solid samples were obtained by filtration. The solid sample was subjected to XRPD testing after vacuum drying at room temperature. The results indicated that free crystal form A was obtained in all solvent systems listed in Table 1-3. The free crystal form A obtained from the solvent EtOH was selected for XRPD, TGA, and DSC tests. Its XRPD pattern is shown in FIG. 1-1, and the XRPD diffraction peak data are shown in Table 1-4; the DSC thermogram is shown in FIG. 1-2, and the TGA thermogram is shown in FIG. 1-3.

**Table 1-3: Polymorph screening results by room temperature slurry method**

| **Solvent System** | **Crystal form** |
|---|---|
| H₂O | Free Crystal Form A |
| EtOH | Free Crystal Form A |
| IPA | Free Crystal Form A |
| EA | Free Crystal Form A |
| IPAc | Free Crystal Form A |
| THF | Free Crystal Form A |
| 1,4-Dioxane | Free Crystal Form A |
| MTBE | Free Crystal Form A |
| Acetone | Free Crystal Form A |
| MEK | Free Crystal Form A |
| DMSO | Free Crystal Form A |
| ACN | Free Crystal Form A |
| ACN/H₂O | Free Crystal Form A |
| Toluene | Free Crystal Form A |
| DMF/ACN | Free Crystal Form A |
| NMP/H₂O | Free Crystal Form A |
| THF/H₂O | Free Crystal Form A |
| acetone/H₂O | Free Crystal Form A |

**Table 1-4: XRPD diffraction peak data of free crystal form A**

| Peak No. | 2θ [°] | Relative intensity [%] |
|---|---|---|
| 1 | 5.777 | 24.00 |
| 2 | 8.101 | 4.60 |
| 3 | 9.055 | 2.80 |
| 4 | 10.771 | 7.50 |
| 5 | 11.564 | 10.10 |
| 6 | 12.854 | 34.60 |
| 7 | 16.198 | 33.20 |
| 8 | 17.386 | 12.30 |
| 9 | 17.793 | 58.00 |
| 10 | 18.134 | 65.60 |
| 11 | 18.454 | 3.70 |
| 12 | 18.925 | 3.30 |
| 13 | 19.547 | 1.60 |
| 14 | 20.515 | 100.00 |
| 15 | 21.630 | 54.70 |
| 16 | 22.010 | 5.80 |
| 17 | 23.195 | 4.60 |
| 18 | 23.887 | 4.70 |
| 19 | 24.455 | 36.60 |
| 20 | 24.997 | 34.10 |
| 21 | 25.555 | 7.00 |
| 22 | 25.969 | 61.10 |
| 23 | 26.386 | 4.20 |
| 24 | 27.334 | 22.70 |
| 25 | 27.766 | 12.80 |
| 26 | 28.832 | 17.50 |
| 27 | 29.034 | 15.10 |
| 28 | 29.599 | 4.80 |
| 29 | 30.333 | 17.70 |
| 30 | 30.617 | 18.70 |
| 31 | 31.142 | 4.40 |
| 32 | 31.770 | 6.80 |
| 33 | 32.225 | 3.10 |
| 34 | 32.862 | 2.60 |
| 35 | 33.326 | 5.30 |
| 36 | 33.765 | 3.10 |
| 37 | 34.248 | 16.60 |
| 38 | 34.642 | 27.10 |
| 39 | 35.189 | 5.60 |
| 40 | 36.006 | 7.10 |
| 41 | 38.663 | 9.10 |

### Example 5: Preparation of polymorphs by 50°C slurry method

Approximately 50 mg of the compound of formula **I** was weighed into separate 2 mL clear vials. 0.1-0.5 mL of the solvents listed in Table 1-5 was added to each vial at 50°C, followed by magnetic stirring for about one week. The solid sample was obtained by filtration. After vacuum drying at room temperature, the samples were subjected to XRPD testing. The results indicated that free crystal form A was obtained in all solvent systems listed in Table 1-5.

**Table 1-5: Polymorph screening results by 50°C slurry method**

| **Solvent System** | **Crystal form** |
|---|---|
| H₂O | Free Crystal Form A |
| EtOH | Free Crystal Form A |
| IPA | Free Crystal Form A |
| EA | Free Crystal Form A |
| IPAc | Free Crystal Form A |
| THF | Free Crystal Form A |
| 1,4-Dioxane | Free Crystal Form A |
| MTBE | Free Crystal Form A |
| Acetone | Free Crystal Form A |
| MEK | Free Crystal Form A |
| DMSO | Free Crystal Form A |
| ACN | Free Crystal Form A |
| ACN/H₂O | Free Crystal Form A |
| Toluene | Free Crystal Form A |
| DMF/ACN | Free Crystal Form A |
| NMP/H₂O | Free Crystal Form A |
| THF/H₂O | Free Crystal Form A |
| acetone/H₂O | Free Crystal Form A |

### Example 6: Preparation of polymorphs by slow evaporation method

Approximately 40 mg of the compound of formula **I** was weighed into separate 2 mL clear vials. 2-7 mL of the solvents listed in Table 1-6 was added to each vial. The resulting solution was filtered through a 0.22 µm filter membrane, and the filtered clear solution was allowed to slowly evaporate at room temperature to afford a solid. After vacuum drying at room temperature, the samples were subjected to XRPD testing. The results indicated that free crystal form A was obtained in all solvent systems listed in Table 1-6. When the solvent was 1,4-dioxane, a mixture of free crystal form B and a small amount of free crystal form A was obtained.

**Table 1-6: Polymorph screening results by slow evaporation method**

| **Solvent System** | **Crystal form** |
|---|---|
| MeOH | Free Crystal Form A |
| EtOH | Free Crystal Form A |
| EA | Free Crystal Form A |
| THF | Free Crystal Form A |
| 1,4-Dioxane | Free crystal form B + a small amount of free crystal form A |
| Acetone | Free Crystal Form A |
| MEK | Free Crystal Form A |
| DCM | Free Crystal Form A |
| ACN | Free Crystal Form A |

### Example 7: Preparation of polymorphs by anti-solvent method

Approximately 40 mg of the compound of formula **I** was weighed and added into separate 2 mL clear vials. The sample was dissolved with a small amount of a good solvent at room temperature, and the resulting solution was filtered through a 0.22 µm filter membrane. Then, 2-4 volumes of anti-solvent were slowly added to the filtered clear solution until solid precipitated. The solid sample was obtained by filtration. After vacuum drying at room temperature, the samples were subjected to XRPD testing. The results showed that under the solvent systems listed in Table 1-7, free crystal form A and free crystal form B were obtained.

**Table 1-7: Polymorph Screening Results by Anti-Solvent Method**

| **Solvent System** | | **Crystal form** |
|---|---|---|
| Good Solvent | Anti-solvent | |
| DMSO | H₂O | Free Crystal Form A |
| DMF | H₂O | Free Crystal Form A |
| DCM | MTBE | Free Crystal Form A |
| THF | H₂O | Free Crystal Form A |
| THF | Heptane | Free crystal form B |
| MEK | Heptane | Free Crystal Form A |
| NMP | H₂O | Free Crystal Form A |
| DMAc | H₂O | Free Crystal Form A |
| EtOH | Heptane | Free Crystal Form A |
| IPAc | Heptane | Free crystal form B |

### Example 8: Preparation of Polymorphs by Reverse Anti-Solvent Method

Approximately 40 mg of the compound of formula **I** was weighed into separate 2 mL clear vials. The sample was dissolved with a small amount of a good solvent at room temperature, and the resulting solution was filtered through a 0.22 µm filter membrane. Then, the filtered clear solution was quickly added to 2-4 volumes of anti-solvent, causing solid to precipitate. The solid sample was obtained by filtration. After vacuum drying at room temperature, the samples were subjected to XRPD testing. The results showed that under the solvent systems listed in Table 1-8, free crystal form A and free crystal form B were obtained. When the good solvent was 1,4-dioxane and the anti-solvent was H₂O, a mixture of free crystal form B and a small amount of free crystal form A was obtained.

**Table 1-8: Polymorph Screening Results by Reverse Anti-Solvent Method**

| **Solvent System** | | **Crystal form** |
|---|---|---|
| Good Solvent | Anti-solvent | |
| DMSO | H₂O | Free Crystal Form A |
| DMF | H₂O | Free Crystal Form A |
| DCM | Heptane | Free crystal form B |
| EA | Heptane | Free Crystal Form A |
| 1,4-Dioxane | Heptane | Free crystal form B |
| 1,4-Dioxane | MTBE | Free Crystal Form A |
| 1,4-Dioxane | H₂O | Free crystal form B + a small amount of free crystal form A |
| MEK | MTBE | Free Crystal Form A |
| MEK | Toluene | Free Crystal Form A |
| THF | Toluene | Free Crystal Form A |

### Example 9: Preparation of Polymorphs by Cooling Method

Approximately 40 mg of the compound of formula **I** was weighed into separate 2 mL clear vials. The sample was dissolved with a small amount of solvent at 50°C, and the resulting solution was filtered through a 0.22 µm membrane filter. The filtered clear solution was allowed to cool slowly to room temperature under magnetic stirring, during which solid precipitated. The solid sample was obtained by filtration, dried under vacuum at room temperature, and then subjected to XRPD testing. The results showed that under the solvent systems listed in Table 1-9, free crystal form A and free crystal form B were obtained.

**Table 1-9: Polymorph Screening Results by Cooling Method**

| **Solvent System** | **Crystal form** |
|---|---|
| MeOH | Free Crystal Form A |
| EtOH | Free Crystal Form A |
| IPA | Free crystal form B |
| IPAc | Free Crystal Form A |
| ACN | Free Crystal Form A |
| EA | Free Crystal Form A |
| Acetone | Free Crystal Form A |

### Example 10: Preparation of Polymorphs by Grinding Method

Approximately 40 mg of the compound of formula **I** was weighed into a mortar and ground for about 10 minutes under different conditions. After the ground solid was dried under vacuum at room temperature, the sample was subjected to XRPD testing. The results showed that under the solvent systems listed in Table 1-10, free crystal form A containing a large amount of amorphous material was obtained.

**Table 1-10: Polymorph Screening Results by Grinding Method**

| **Solvent System** | **Crystal form** |
|---|---|
| NA | Free crystal form A, containing a large amount of amorphous form |
| H₂O | Free crystal form A, containing a large amount of amorphous form |
| MTBE | Free crystal form A, containing a large amount of amorphous form |
| Heptane | Free crystal form A, containing a large amount of amorphous form |

### Example 11: Preparation of Free Crystal Form B

The preparation process of free crystal form B is as follows:
500 mg of the compound of formula I was weighed into a round-bottom flask. 6.25 mL of THF was added to the round-bottom flask at room temperature, and the sample was dissolved by sonication. The resulting solution was filtered through a 0.22 µm membrane filter into another 100 mL round-bottom flask. Under magnetic stirring, 18.75 mL of heptane was slowly added to the filtered clear solution, causing white solid to precipitate. Subsequently, about 5 mg of seed crystals of free crystal form B (obtained from the anti-solvent method using THF/heptane in Example 7) was added, and stirring was continued. The mixture was stirred for about three hours at room temperature, then filtered under vacuum to obtain a white solid. The solid was dried under vacuum at room temperature and then subjected to XRPD, TGA, and DSC testing. The XRPD pattern of free crystal form B is shown in FIG. 2-1, and the XRPD diffraction peak data are shown in Table 1-12; the DSC thermogram is shown in FIG. 2-2, and the TGA thermogram is shown in FIG. 2-3.

**Table 1-11: Characterization of free crystal form B**

| **XRPD** | **DSC endothermic peak temperature/°C** | **TGA/%** |
|---|---|---|
| Free crystal form B | 168.47 | 0.2289 (up to 150 °C) |

**Table 1-12: XRPD diffraction peak data of free crystal form B of Example 11**

| Peak No. | 2θ [°] | Relative intensity [%] |
|---|---|---|
| 1 | 5.797 | 100.00 |
| 2 | 11.641 | 38.40 |
| 3 | 12.599 | 39.50 |
| 4 | 16.086 | 8.20 |
| 5 | 17.456 | 98.90 |
| 6 | 18.365 | 9.40 |
| 7 | 20.930 | 89.80 |
| 8 | 22.189 | 14.40 |
| 9 | 23.029 | 6.20 |
| 10 | 23.815 | 8.60 |
| 11 | 25.156 | 57.40 |
| 12 | 26.562 | 37.50 |
| 13 | 28.218 | 16.50 |
| 14 | 28.511 | 19.30 |
| 15 | 29.251 | 17.10 |
| 16 | 29.794 | 7.80 |
| 17 | 32.203 | 6.80 |
| 18 | 33.229 | 10.80 |
| 19 | 34.423 | 7.80 |
| 20 | 35.320 | 12.70 |
| 21 | 38.520 | 13.60 |

### Example 12: Salt form screening

Using a sample of free crystal form A as the starting material, 39 salt form screening experiments were set up by selecting 13 ligands (with an acid-base feed ratio of 1:1) and 3 solvents. The specific steps of the screening experiments were as follows: approximately 20 mg of free crystal form A sample and an equimolar amount of the corresponding ligand were weighed into an HPLC vial, 0.5 mL of solvent was added and mixed to obtain a suspension; liquid acids were first diluted with the corresponding solvent before being mixed with the starting sample. The mixtures were suspended and stirred at room temperature for about 3 days, centrifuged to separate the solids, and then dried under vacuum at 50 °C. XRPD characterization results of the obtained solids (Table 2-1) showed that a total of 2 salt form crystal samples were obtained in the salt form screening experiments, namely maleate salt crystal form A and sodium salt crystal form A. The salt form samples obtained by screening were subjected to TGA/DSC characterization, and the salt-forming molar ratios were determined by ¹H NMR and HPLC/IC. The salt form characterization results are summarized in Table 2-2.

**Table 2-1. Summary of salt form screening test results**

| **No.** | **Ligand*** | **A IPA** | **B EtOAc** | **C 2-MeTHF** |
|---|---|---|---|---|
| 0. | Blank | Free Crystal Form A | Free Crystal Form A | Free Crystal Form A |
| 1. | Hydrochloric acid | Free Crystal Form A | Hydrochloride salt crystal form A^{abd#} (53.25% area) | Gelation ^{abc} |
| 2. | Sulfuric acid | Free Crystal Form A | Gel formation^{abc#} (46.91% area) | Gel formation^{abc} |
| 3. | Maleic acid | Free Crystal Form A | Crystal form A of maleate | Free Crystal Form A |
| 4. | Phosphoric acid | Free Crystal Form A | Free crystal form A^{a} | Free crystal form A^{ab} |
| 5. | Fumaric acid | Free Crystal Form A | Free Crystal Form A + Fumaric Acid | Free Crystal Form A |
| 6. | Tartaric acid | Free Crystal Form A | Free Crystal Form A | Free Crystal Form A |
| 7. | Citric Acid | Free Crystal Form A | Free crystal form A^{a} | Free crystal form A^{a} |
| 8. | L-Malic Acid | Free Crystal Form A | Free Crystal Form A | Free Crystal Form A |
| 9. | Succinic Acid | Free Crystal Form A + Succinic Acid | Free Crystal Form A + Succinic Acid | Free Crystal Form A + Succinic Acid |
| 10. | p-Toluenesulfonic acid | Free Crystal Form A | Gelation^{abde} | Gelation^{abc} |
| 11. | Methanesulfonic acid | Free Crystal Form A | Gelation^{abde#} (27.88% area) | Gelation^{abc} |
| 12. | Sodium Hydroxide | Sodium Salt Crystal Form A^{abd} | Sodium Salt Crystal Form A | Sodium Salt Crystal Form A |
| 13. | Arginine | Free Crystal Form A + Arginine | Arginine | Free Crystal Form A + Arginine |

| | | | | |
|---|---|---|---|---|
| *: The molar feed ratio of ligand to free form was 1:1; ^{#}: The sample was observed to turn orange-red. ^{a}: The sample became clear after stirring at room temperature, then stirred at 5 °C; ^{b}: The sample became clear after stirring at 5 °C, then 1.0 mL of n-heptane was added to induce crystallization; ^{c}: The sample formed a gel after adding n-heptane to induce crystallization, then subjected to temperature cycling between 50 °C and 5 °C; ^{d}: The sample remained clear after adding n-heptane to induce crystallization, then stirred at 5 °C; ^{e}: The sample remained clear after stirring at 5 °C, then allowed to evaporate at room temperature. | | | | |

**Table 2-2. Summary of characterization results of salt forms obtained by screening**

| **Salt form** | **TGA weight loss (%)** | **DSC endothermic signal (°C, peak temperature)** | **Molar ratio# (acid/base)** | **Solvent residue# (wt%)** |
|---|---|---|---|---|
| Crystal form A of maleate | 1.41 (120 °C) | 139.1 | 1.0 | ND |
| Sodium Salt Crystal Form A | 2.75 (150 °C) | 191.0, 215.0 | 1.0 | ND |

| | | | | |
|---|---|---|---|---|
| ^{#} : The ratio or value was obtained by ¹H NMR or HPLC/IC calculation; ND: not detected. | | | | |

### 12.1Maleate Crystal Form A

Maleate crystal form A was obtained by slurrying a sample of free crystal form A with an equimolar amount of maleic acid in EtOAc at room temperature for 3 days, centrifuging to isolate the solid sample, and drying under vacuum at 50 °C. The XRPD and TGA/DSC results for the maleate crystal form A sample are shown in Figure 3-1 and Figure 3-2, respectively. The TGA results show a weight loss of 1.41% when the sample was heated to 120 °C. The DSC results show an endothermic signal was observed for the sample at 139.1 °C (onset temperature). ¹H NMR results, as shown in FIG. 3-3, show that the molar ratio of maleic acid to the compound of formula I in the sample is 1:1, and no EtOAc solvent residue was found. The XRPD diffraction peak data of crystal form A of maleate are shown in Table 2-3 below.

**Table 2-3. XRPD diffraction peak data of crystal form A of maleate**

| Peak No. | 2θ[°] | Relative intensity [%] |
|---|---|---|
| 1 | 8.3940 | 71.26 |
| 2 | 10.6516 | 1.33 |
| 3 | 13.7796 | 30.77 |
| 4 | 16.3059 | 100.00 |
| 5 | 17.0650 | 53.93 |
| 6 | 18.4439 | 48.69 |
| 7 | 19.0068 | 4.19 |
| 8 | 20.4460 | 14.51 |
| 9 | 20.9626 | 29.75 |
| 10 | 21.6068 | 20.93 |
| 11 | 22.6483 | 8.73 |
| 12 | 23.1940 | 11.30 |
| 13 | 24.6212 | 6.46 |
| 14 | 25.2886 | 20.83 |
| 15 | 25.6193 | 21.81 |
| 16 | 26.1891 | 18.70 |
| 17 | 26.7780 | 22.49 |
| 18 | 27.0341 | 9.02 |
| 19 | 27.4148 | 4.28 |
| 20 | 28.4710 | 8.98 |
| 21 | 28.9586 | 10.32 |
| 22 | 30.0525 | 5.35 |
| 23 | 30.7434 | 2.80 |
| 24 | 31.2511 | 5.49 |
| 25 | 31.6144 | 6.52 |
| 26 | 32.6983 | 3.57 |
| 27 | 33.1269 | 2.82 |
| 28 | 33.9061 | 7.56 |
| 29 | 34.4874 | 3.99 |
| 30 | 35.2211 | 3.62 |
| 31 | 36.2144 | 2.36 |
| 32 | 38.4818 | 2.62 |

### 12.2 Preparation of sodium salt crystal form A

Sodium salt crystal form A was obtained by slurrying a sample of free crystal form A with an equimolar amount of sodium hydroxide in 2-MeTHF at room temperature for 3 days, centrifuging to separate the solid sample, and drying under vacuum at 50 °C. The XRPD and TGA/DSC results for the sodium salt crystal form A sample are shown in FIG. 4-1 and FIG. 4-2, respectively. The TGA results show that the sample had a weight loss of 2.75% when heated to 150 °C, and the DSC results show that the sample had 2 endothermic peaks at 191.0 °C and 215.0 °C (peak temperatures). ¹H NMR results, as shown in FIG. 4-3, show that no 2-MeTHF solvent residue was found in the sample. HPLC/IC results show that the molar ratio of Na⁺ to the compound of formula I in sodium salt crystal form A is 1:1. The XRPD diffraction peak data of sodium salt crystal form A are shown in Table 2-4 below.

**Table 2-4. XRPD diffraction peak data of sodium salt crystal form A**

| **Peak No.** | **2θ[°]** | **Relative intensity [%]** |
|---|---|---|
| 1 | 4.8516 | 11.52 |
| 2 | 9.7049 | 9.10 |
| 3 | 12.1335 | 10.25 |
| 4 | 13.5513 | 12.70 |
| 5 | 14.2041 | 10.56 |
| 6 | 14.5497 | 11.30 |
| 7 | 16.6950 | 42.03 |
| 8 | 17.0192 | 59.41 |
| 9 | 17.6693 | 18.53 |
| 10 | 18.5056 | 22.50 |
| 11 | 20.2736 | 8.46 |
| 12 | 21.2287 | 51.72 |
| 13 | 22.3258 | 100.00 |
| 14 | 23.3504 | 5.50 |
| 15 | 24.3947 | 31.55 |
| 16 | 25.5021 | 63.25 |
| 17 | 25.8915 | 72.27 |
| 18 | 26.7174 | 12.97 |
| 19 | 27.7399 | 9.94 |
| 20 | 28.4352 | 10.81 |
| 21 | 28.8693 | 3.50 |
| 22 | 29.7328 | 21.56 |
| 23 | 30.7610 | 13.35 |
| 24 | 31.4308 | 8.82 |
| 25 | 31.8816 | 6.88 |
| 26 | 32.4553 | 6.21 |
| 27 | 32.9794 | 2.04 |
| 28 | 34.0556 | 1.30 |
| 29 | 35.1324 | 3.91 |
| 30 | 35.6774 | 2.59 |
| 31 | 36.3298 | 3.57 |
| 32 | 38.2993 | 3.40 |
| 33 | 39.7133 | 7.13 |

### Example 13 Evaluation Test

### 13.1 Dynamic Solubility

Solids at a feeding concentration of 10 mg/mL (calculated based on free crystal form) were rotationally mixed with corresponding vehicles at 37 °C. The solubility of each sample in H₂O, SGF, FaSSIF, and FeSSIF was determined at different time points (1 h, 2 h, 4 h, and 24 h). After sampling at each time point, the samples were centrifuged (at 10000 rpm) and filtered (through 0.45 µm PTFE), the HPLC concentrations and pH values of the filtrates were determined, and the centrifuged solid samples were subjected to XRPD analysis. The solubility test results are summarized in Table 2-5, and the solubility curves are shown in FIG. 5. XRPD results show that the crystal form of free crystal form A remained unchanged after the dynamic solubility test (FIG. 6-1 to FIG. 6-4).

**Table 2-5. Summary of dynamic solubility test results at 37 °C**

| **Starting material** | **Vehicle** | **1 h** | **2 h** | **4 h** | **24 h** |
|---|---|---|---|---|---|
| | | S | S | S | S |
| Free Crystal Form A | H₂O | 0.002 | 0.001 | 0.002 | 0.002 |
| | SGF | 0.012 | 0.012 | 0.013 | 0.013 |
| | FaSSIF | 0.013 | 0.014 | 0.015 | 0.015 |
| | FeSSIF | 0.051 | 0.053 | 0.055 | 0.059 |
| Crystal form A of maleate | H₂O | 0.006 | 0.001 | 0.001 | 0.002 |
| | SGF | 0.001 | 0.006 | 0.006 | 0.007 |
| | FaSSIF | 0.009 | 0.019 | 0.031 | 0.009 |
| | FeSSIF | 0.060 | 0.062 | 0.059 | 0.057 |

| | | | | | |
|---|---|---|---|---|---|
| S: solubility (mg/mL). | | | | | |

### 13.2 Hygroscopicity

Hygroscopicity evaluation of free crystal form A and crystal form A of maleate was performed using a dynamic vapor sorption (DVS) instrument. The percentage changes in mass of the samples when the humidity changed (0% RH to 95% RH) at a constant temperature of 25 °C were collected. The DVS test results and XRPD results for the samples before and after the DVS test are shown in FIG. 7-1 to FIG. 7-4. The results show that the moisture adsorption of free crystal form A and crystal form A of maleate at 25 °C/80% RH was 0.1698% and 0.224%, respectively; all samples showed no change in crystal form after the DVS test, and in particular, free crystal form A exhibited almost no hygroscopicity.

### 13.3 Solid State Stability

Free crystal form A and the maleate salt crystal form A were placed at 80 °C/closed for one day and at 25 °C/60% RH and 40 °C/75% RH/open for one week, respectively. The physical and chemical stability of the samples were tested by XRPD and HPLC. Purity data are listed in Table 2-6, and XRPD results are shown in Figures 8-1 and 8-2. The results showed no significant change in purity and no change in crystal form for all samples.

**Table2-6 Summary of Solid State Stability Evaluation**

| **Crystal form** | **Conditions** | | **HPLC results** | | |
|---|---|---|---|---|---|
| | | | P̅u̅r̅i̅t̅y̅ (area%) | Relative to starting (%) | Crystal form |
| Free Crystal Form A | Starting | | 99.39 | -- | -- |
| | 25 °C/60% RH/open | 1 week | 99.35 | 100.0 | |
| | 40 °C/75% RH/open | 1 week | 99.37 | 100.0 | Free Crystal Form A |
| | 80 °C/sealed | 1 day | 99.45 | 100.1 | |
| Crystal form A of maleate | Starting | | 99.94 | -- | -- |
| | 25 °C/60% RH/open | 1 week | 99.90 | 100.0 | |
| | 40 °C/75% RH/open | 1 week | 99.95 | 100.0 | C rystal form A of maleate |
| | 80 °C/sealed | 1 day | 99.93 | 100.0 | |

### Test Example 1: Detection of Inhibitory Activity of Compounds on Nav1.8 Ion Channel

Reagents, except NaOH and KOH used for acid-base titration, were purchased from Sigma (St. Louis, MO). The final concentrations of test compounds were prepared on the day of testing and then dissolved in extracellular fluid. The extracellular fluid (mM) consisted of: NaCl, 137 mM; KCl, 4 mM; CaCl₂, 1.8 mM; MgCl₂, 1 mM; HEPES, 10 mM; glucose, 10 mM; pH 7.4 (titrated with NaOH). All test compound and control compound solutions contained 1 µM TTX. The intracellular fluid (mM) consisted of: aspartic acid, 140 mM; magnesium chloride, 2 mM; ethylene glycol tetraacetic acid (EGTA), 11 mM; N-2-hydroxyethylpiperazine-N'-2-ethanesulfonic acid (HEPES), 10 mM. The pH was adjusted to 7.4 with cesium hydroxide.

Test compounds were dissolved in dimethyl sulfoxide (DMSO) at a concentration of 9 mM. On the day of testing, they were further dissolved in extracellular fluid to the required concentrations.

### Electrophysiological Experimental Procedure:

The cells were transferred to a perfusion chamber and perfused with extracellular fluid. The intracellular fluid was thawed on the day of the experiment. Electrodes were pulled using a PC-10 puller (Narishige, Japan). Whole-cell patch-clamp recordings were performed, and noise was filtered at one-fifth of the sampling frequency. One-quarter of the electrode barrel length was filled with intracellular fluid, and the electrode was mounted on the probe. The required protocol was set up, the interface was switched to Membrane test, and the Stage was set to Bath. Positive pressure was applied inside the electrode, and the electrode tip was brought into contact with a cell. The three-way valve of the suction device was turned to the open position, and then negative pressure was applied to the electrode to form a high-resistance seal between the electrode and the cell. The Stage was set to Patch, the leak was controlled within -200 pA, and negative pressure was continued to be applied to rupture the cell membrane and establish a current pathway. The suction device and the extracellular fluid valve were opened to begin perfusion. The cell current was observed, and drug administration was started after the cell current stabilized (at least 3 sweep current curves overlapped). The compound was administered from low to high concentrations, with the perfusion time for each concentration being no less than 2 minutes, and the concentration should be changed after the current stabilized.

The test article was administered via a gravity-fed perfusion system. During the initial recording period, the peak current amplitude was observed for at least 1 minute until it stabilized. During this period, the CV% of all peak current amplitudes should be less than 10% to exclude significant fluctuations in the initial current. The average of the peak current amplitudes from the last 10 recordings during the initial recording period was used as the peak current for the negative control. After the initial current stabilized, the test sample starting from the low concentration was administered until the peak current from 10 recordings stabilized again, or after continuous perfusion for 5 minutes, the peak current remained "unchanged" compared to before administration. We define the following two scenarios as "stable" or "unchanged": 1) if the absolute average of the peak currents from 10 consecutive sweeps exceeds 200 pA with a CV value less than 10%, or 2) the average of the peak currents from 10 consecutive sweeps is between 200 pA and 50 pA with a CV value less than 30%. Then the next higher concentration was administered for testing.

The average of the peak currents from the last 10 sweeps at each concentration was used as the peak current for that concentration for data analysis. If a stable state cannot be reached within 5 minutes, the average of the peak currents from the last 10 sweeps at that time was used as the peak current for that concentration for data analysis. Simultaneously, that cell was to be discarded and not used for testing higher concentrations. Each concentration of the compound was tested on at least two cells.

### Voltage pulse protocol:

The cell was clamped at -80 mV, then depolarized to 10 mV using a 10-millisecond square wave to elicit NaV1.8 currents. This protocol was repeated every 5 seconds. The maximum current elicited by the square wave was detected; after it stabilized, the test compound was perfused, and when the response stabilized, the degree of blockade was calculated.

### Data processing and fitting

Data acquisition and analysis are performed using pCLAMP 10 (Molecular Devices, Union City, CA). Current stabilization refers to the current changing within a limited range over time. The inhibitory activity (IC₅₀) of the drug on the Nav1.8 ion channel is calculated by plotting the dose-response relationship between the serially diluted concentrations of the drug and the stabilized current values produced on HEK293/Nav1.8 cells.

**Table B-1: Inhibitory Activity of Compounds on the Nav1.8 Ion Channel**

| Compound | IC₅₀ (nM) |
|---|---|
| I | 14 |

The test results indicate that the compounds of the present disclosure exhibit strong inhibitory activity on the Nav1.8 ion channel.

### Test Example 2: Mouse Pharmacokinetic Study

In the mouse pharmacokinetic study, three male ICR mice were fasted overnight and administered a single oral dose of 10 mg/kg by gavage. Blood samples were collected before administration and at 15 and 30 minutes, and 1, 2, 4, 6, 8, and 24 hours after administration. The blood samples were centrifuged at 8000 rpm for 6 minutes at 4°C, and the plasma was collected and stored at -20°C. Plasma from each time point was taken, mixed with 3-5 volumes of acetonitrile solution containing an internal standard, vortexed for 1 minute, and centrifuged at 13000 rpm for 10 minutes at 4°C. The supernatant was taken, mixed with 3 volumes of water, and an appropriate amount of the mixture was subjected to LC-MS/MS analysis. The main pharmacokinetic parameters were analyzed using the non-compartmental model in WinNonlin 7.0 software.

**Table B-2: Results of Mouse Pharmacokinetic Study for the Compounds**

| Compound Number | Mouse Pharmacokinetic Parameters | | | |
|---|---|---|---|---|
| | Oral Administration (10 mg/kg) | | | |
| | Cmax (ng/mL) | Tmax (hr) | AUC0-t (h*ng/mL) | T_{1/2} (h) |
| I | 7370 | 0.25 | 17273 | 2.74 |

| | | | | |
|---|---|---|---|---|
| The test results indicate that the compounds of the present disclosure possess favorable pharmacokinetic characteristics. | | | | |

### Test Example 3: Rat Spinal Nerve Ligation Neuropathic Pain Model

Male SD rats weighing 180-220 g were anesthetized and placed in a prone position on the surgical table. An incision was made along the spine near the animal's hip bone to separate the fascia and muscle. The L5 transverse process was carefully bitten off with forceps, and the L5 nerve was separated using a glass dissecting needle. The L5 nerve was ligated with a 5-0 ligature. The muscle and skin were sutured, and the area was disinfected with iodophor. Fourteen days after modeling, the animals were divided into different groups (10 animals per group) and orally administered different compounds via gavage. The mechanical pain threshold of the animals was measured using Von-Frey filaments at different time points after administration. Specific dosing regimens and detection time points are detailed in Table B-3 below.

Mechanical pain threshold detection method: The test animal was continuously stimulated on the plantar surface of the hind paw to be tested with a Von-Frey filament until the filament bent, and the paw withdrawal response of the animal was observed. The test animal was stimulated sequentially with filaments of increasing gram force, from the smallest to the largest. Each filament gram force was applied five consecutive times. If the number of positive responses was less than 3, the procedure was repeated with the next larger filament. The first filament gram force that elicited 3 or more positive responses was recorded as the pain threshold for that animal (each animal was tested three times, and the average value was taken). Filament gram forces: 0.6, 1.0, 1.4, 2.0, 4.0, 6.0, 8.0, 10.0, 15.0; cutoff value: 15.0 g.

**Table B-3: Efficacy of Compounds on Pain Threshold in Rats with Spinal Nerve Ligation**

| No. | Mechanical Pain Threshold (Mean±SD) | | | |
|---|---|---|---|---|
| | Baseline Before Modeling | 1h After Administration | 3h After Administration | 6h After Administration |
| Vehicle | 13.9±2.0 | 4.3±2.0 | 4.4±1.9 | 5.0±1.8 |
| I-100mpk | 13.6±1.7 | 11.0±4.0*** | 11.1±3.7*** | NA |
| Pregabalin-30mpk | 13.6±2.7 | 13.8±2.3*** | 12.3±2.9*** | 12.1±2.3*** |

| | | | | |
|---|---|---|---|---|
| One-way ANOVA; ***P<0.001, **P<0.01, *P<0.05 NA: No test was scheduled at this time point. | | | | |

The test results indicate that the compound of the present disclosure can significantly improve the reduction in mechanical pain threshold in animals induced by rat spinal nerve ligation modeling, demonstrating excellent analgesic efficacy.

### Test Example 4: Rat Incisional Pain Model

Male SD rats weighing 200-250 g were anesthetized and fixed in a prone position. The plantar surface of the lateral hind paw was extended upward, and the toes were fixed with surgical tape, followed by disinfection. A longitudinal incision of approximately 1 cm was made in the skin and fascia using a scalpel, starting 0.5 cm from the heel towards the toes on the animal's plantar surface. After lifting the flexor digitorum brevis muscle with surgical curved forceps, a longitudinal incision was made in the belly of the muscle using a scalpel, without completely transecting the muscle. The skin was sutured and disinfected. On the second day after modeling, the animals were divided into different groups, with 8 animals per group, and were orally administered different compounds by gavage. The mechanical pain threshold of the animals was measured using Von Frey filaments at different time points after administration. The specific group dosing regimens and test time points are detailed in Table B-4 below.

Mechanical pain threshold detection method: The test animal was continuously stimulated on the plantar surface of the hind paw to be tested with a Von-Frey filament until the filament bent, and the paw withdrawal response of the animal was observed. The test animal was stimulated sequentially with filaments of increasing gram force, from the smallest to the largest. Each filament gram force was applied five consecutive times. If the number of positive responses was less than 3, the procedure was repeated with the next larger filament. The first filament gram force that elicited 3 or more positive responses was recorded as the pain threshold for that animal (each animal was tested three times, and the average value was taken). Filament gram forces: 0.6, 1.0, 1.4, 2.0, 4.0, 6.0, 8.0, 10.0, 15.0; cutoff value: 15.0 g.

**Table B-4: Efficacy of Compounds on Pain Threshold in Incisional Pain Model Rats**

| No. | Mechanical Pain Threshold (Mean±SD) | | | |
|---|---|---|---|---|
| | Baseline Before Modeling | 1h After Administration | 3h After Administration | 6h After Administration |
| Vehicle | 12.6±1.9 | 2.5±0.5 | 2.9±0.6 | 2.8±0.7 |
| I-100mpk | 12.6±1.7 | 12.5±1.9*** | 11.4±2.0*** | 8.4±1.2*** |
| Pregabalin-30mpk | 12.7±1.7 | 12.4±1.9*** | 11.6±1.1*** | 11.3±1.4*** |

| | | | | |
|---|---|---|---|---|
| One-way ANOVA, vs. Vehicle group, ***P<0.001 | | | | |

The experimental results indicate that the compound of the present disclosure can significantly improve the reduction in mechanical pain threshold in animals induced by the rat incisional pain model, demonstrating excellent analgesic efficacy.

## Claims

1. A crystal form of a compound of formula I or a pharmaceutically acceptable salt thereof, wherein the compound of formula I has the following structure:

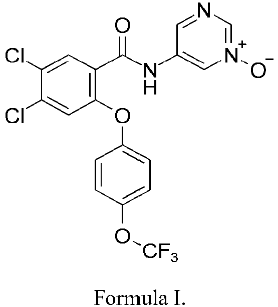

2. The crystal form according to claim 1, wherein the crystal form is a free crystal form A of the compound of formula I, and the free crystal form A has an X-ray powder diffraction pattern comprising diffraction peaks at 2θ±0.2° diffraction angles of 17.79°, 18.13°, 20.52°, 21.63°, and 25.97°;
preferably, the free crystal form A has an X-ray powder diffraction pattern further comprising diffraction peaks at 2θ±0.2° diffraction angles of one or more of 12.85°, 16.20°, 24.46°, and 25.00°;
preferably, the free crystal form A has an X-ray powder diffraction pattern comprising diffraction peaks at 2θ±0.2° diffraction angles of 12.85°, 16.20°, 17.79°, 18.13°, 20.52°, 21.63°, 24.46°, 25.00°, and 25.97°;
preferably, the free crystal form A has an X-ray powder diffraction pattern comprising diffraction peaks at 2θ±0.2° diffraction angles of 5.78°, 11.56°, 12.85°, 16.20°, 17.39°, 17.79°, 18.13°, 20.52°, 21.63°, 24.46°, 25.00°, 25.97°, 27.33°, 27.77°, 28.83°, 29.03°, 30.33°, 30.62°, 34.25°, and 34.64°;
preferably, the free crystal form A has an X-ray powder diffraction pattern comprising diffraction peaks at 2θ±0.2° diffraction angles of 5.78°, 10.77°, 11.56°, 12.85°, 16.20°, 17.39°, 17.79°, 18.13°, 20.52°, 21.63°, 22.01°, 24.46°, 25.00°, 25.56°, 25.97°, 27.33°, 27.77°, 28.83°, 29.03°, 30.33°, 30.62°, 31.77°, 33.33°, 34.25°, 34.64°, 35.19°, 36.01°, and 38.66°;
preferably, the free crystal form A has an X-ray powder diffraction pattern comprising diffraction peaks at 2θ±0.2° diffraction angles of 5.78°, 8.10°, 9.06°, 10.77°, 11.56°, 12.85°, 16.20°, 17.39°, 17.79°, 18.13°, 18.45°, 18.93°, 19.55°, 20.52°, 21.63°, 22.01°, 23.20°, 23.89°, 24.46°, 25.00°, 25.56°, 25.97°, 26.39°, 27.33°, 27.77°, 28.83°, 29.03°, 29.60°, 30.33°, 30.62°, 31.14°, 31.77°, 32.23°, 33.33°, 33.77°, 34.25°, 34.64°, 35.19°, 36.01°, 38.66°, and 32.86°;
preferably, the free crystal form A has an XRPD pattern substantially as shown in FIG. 1-1;
preferably, the free crystal form A is an anhydrate;
preferably, the free crystal form A has one, two, or three of the following characteristics:
(1) a TGA curve of the free crystal form A showing a weight loss of about 0.38±1% at 150.0+3 °C;
(2) a DSC curve of the free crystal form A having a starting point of an endothermic peak at 169.5±3 °C; and
(3) a DSC curve of the free crystal form A having an endothermic peak at 171.0+3 °C;
preferably, the DSC diagram of the free crystal form A is substantially as shown in FIG. 1-2;
preferably, the TGA diagram of the free crystal form A is substantially as shown in FIG. 1-3.

3. The crystal form according to claim 1, wherein the crystal form is a free crystal form B of the compound of formula I, and the free crystal form B has an X-ray powder diffraction pattern comprising diffraction peaks at 2θ±0.2° diffraction angles of 11.64°, 12.60°, 17.46°, 20.93°, 25.16°, and 26.56°;
preferably, the free crystal form B has an X-ray powder diffraction pattern further comprising diffraction peaks at 2θ±0.2° diffraction angles of one or more of 5.80°, 29.25°, 28.22°, 28.51°, and 35.32°;
preferably, the free crystal form B has an X-ray powder diffraction pattern comprising diffraction peaks at 2θ±0.2° diffraction angles of 5.80°, 11.64°, 12.60°, 17.46°, 20.93°, 22.19°, 25.16°, 26.56°, 28.22°, 28.51°, 29.25°, 33.23°, 35.32°, and 38.52°;
preferably, the free crystal form B has an X-ray powder diffraction pattern comprising diffraction peaks at 2θ±0.2° diffraction angles of 5.80°, 11.64°, 12.60°, 16.09°, 17.46°, 18.37°, 20.93°, 22.19°, 23.03°, 23.82°, 25.16°, 26.56°, 28.22°, 28.51°, 29.25°, 29.79°, 32.20°, 33.23°, 34.42°, 35.32°, and 38.52°;
preferably, the free crystal form B has an XRPD pattern substantially as shown in FIG. 2-1;
preferably, the free crystal form B is an anhydrous crystal form;
preferably, the free crystal form B has one, two, or three of the following characteristics:
(1) a TGA curve of the free crystal form B showing a weight loss of about 0.23±1% at 150.0±3 °C;
(2) a DSC curve of the free crystal form B having a starting point of an endothermic peak at 165.8±3 °C; and
(3) a DSC curve of the free crystal form B having an endothermic peak at 168.47±3 °C;
preferably, the DSC diagram of the free crystal form B is substantially as shown in FIG. 2-2;
preferably, the TGA diagram of the free crystal form B is substantially as shown in FIG. 2-3.

4. A method for preparing the free crystal form A of the compound of formula I according to claim 2, wherein any one of the following methods is selected:
Method 1: placing a first sample vial containing the compound of formula I in an open state into a second sample vial containing a solvent, sealing the second sample vial, and allowing it to stand at room temperature; wherein the solvent does not submerge the mouth of the first sample vial;
wherein the solvent is selected from one or more of ethanol, acetone, methyl tert-butyl ether, ethyl acetate, dichloromethane, tetrahydrofuran, acetonitrile, n-heptane, and toluene;
Method 2: placing a first sample vial containing a solution of the compound of formula I in an open state in a second sample vial containing an anti-solvent, sealing the second sample vial, and allowing it to stand at room temperature; wherein the anti-solvent does not submerge the mouth of the first sample vial;
wherein the solvent in the solution of the compound of formula I is selected from one or more of dichloromethane, tetrahydrofuran, 1,4-dioxane, dimethyl sulfoxide, and N,N-dimethylformamide;
and the anti-solvent is selected from one or more of n-heptane, methyl tert-butyl ether, and water;
Method 3: adding a solvent to the compound of formula I at room temperature, stirring magnetically, and collecting the solid;
wherein the solvent is selected from one or more of water, ethanol, isopropanol, ethyl acetate, isopropyl acetate, tetrahydrofuran, 1,4-dioxane, methyl tert-butyl ether, acetone, methyl ethyl ketone, dimethyl sulfoxide, acetonitrile, toluene, N,N-dimethylformamide, and N-methylpyrrolidone;
Method 4: adding a solvent to the compound of formula I at 50°C, stirring magnetically, and collecting the solid;
wherein the solvent is selected from one or more of water, ethanol, isopropanol, ethyl acetate, isopropyl acetate, tetrahydrofuran, 1,4-dioxane, methyl tert-butyl ether, acetone, methyl ethyl ketone, dimethyl sulfoxide, acetonitrile, toluene, N,N-dimethylformamide, and N-methylpyrrolidone;
Method 5: adding the compound of formula I to an organic solvent I, dissolving, then filtering, and volatilizing at room temperature;
preferably, the organic solvent I is selected from one or more of methanol, ethanol, ethyl acetate, tetrahydrofuran, 1,4-dioxane, acetone, methyl ethyl ketone, dichloromethane, and acetonitrile;
Method 6: completely dissolving the compound of formula I in a good solvent, filtering, and adding an anti-solvent dropwise to the clear solution until a solid precipitates;
The good solvent is selected from dimethyl sulfoxide, N,N-dimethylformamide, dichloromethane, tetrahydrofuran, methyl ethyl ketone, N-methylpyrrolidone, N,N-dimethylacetamide, ethanol, and isopropyl acetate;
wherein the anti-solvent is selected from one or more of water, toluene, methyl tert-butyl ether, and n-heptane;
Preferably, when the anti-solvent is selected from water, the good solvent is selected from one of dimethyl sulfoxide, N,N-dimethylformamide, tetrahydrofuran, N-methylpyrrolidone, and N,N-dimethylacetamide; when the anti-solvent is selected from methyl tert-butyl ether, the good solvent is selected from dichloromethane; when the anti-solvent is selected from n-heptane, the good solvent is selected from methyl ethyl ketone or ethanol;
Method 7: completely dissolving the compound of formula I in a good solvent, filtering, and adding the clear solution to an anti-solvent;
wherein the good solvent is selected from one or more of dimethyl sulfoxide, N,N-dimethylformamide, dichloromethane, ethyl acetate, 1,4-dioxane, methyl ethyl ketone, and tetrahydrofuran;
and the anti-solvent is selected from one or more of water, n-heptane, methyl tert-butyl ether, and toluene;
Method 8: adding the compound of formula I to an organic solvent I at 50°C, dissolving, filtering, magnetically stirring, and cooling to room temperature;
preferably, the organic solvent I is selected from one or more of methanol, ethanol, isopropyl acetate, acetonitrile, ethyl acetate, and acetone;
Method 9: adding the compound of formula I to a mortar, or adding the compound of formula I and a solvent to a mortar, and grinding;
preferably, the solvent is selected from one or more of water, methyl tert-butyl ether, and n-heptane.

5. The preparation method of the free crystal form B of the compound of formula I according to claim 3, comprising several methods as follows:
Method 1: completely dissolving the compound of formula I in a good solvent, filtering, and dropwise adding an anti-solvent to the clear solution until a solid precipitates;
wherein the good solvent is selected from one or two of tetrahydrofuran and N,N-dimethylacetamide, and the anti-solvent is selected from n-heptane;
and the anti-solvent is selected from one or more of water, toluene, methyl tert-butyl ether, and n-heptane;
Method 2: completely dissolving the compound of formula I in a good solvent, filtering, and adding the clear solution to an anti-solvent;
wherein the good solvent is selected from one or two of dichloromethane and 1,4-dioxane; the anti-solvent is selected from n-heptane;
Method 3: adding the compound of formula I to isopropanol at 40-60°C (e.g., 50°C), dissolving, filtering, stirring, and cooling to room temperature;
Method 4: completely dissolving the compound of formula I in tetrahydrofuran, filtering, adding n-heptane to the clear solution under stirring until a solid precipitates, adding seed crystals of the free crystal form B, and stirring.

6. A pharmaceutically acceptable salt of a compound of formula I, wherein the structure of the compound of formula I is as follows: 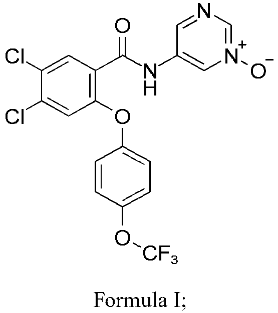
the pharmaceutically acceptable salt is a salt formed from the compound of formula I with an acid or a base;
preferably, the pharmaceutically acceptable salt of the compound of formula I is a salt formed from the compound of formula I and the following acid or base: hydrochloric acid, sulfuric acid, maleic acid, phosphoric acid, fumaric acid, tartaric acid, citric acid, L-malic acid, succinic acid, p-toluenesulfonic acid, methanesulfonic acid, sodium hydroxide, and arginine;
preferably, the pharmaceutically acceptable salt of the compound of formula I is maleate of the compound of formula I; in the maleate, the molar ratio of the compound of formula I to maleic acid is 1:1;
preferably, the pharmaceutically acceptable salt of the compound of formula I is sodium salt of the compound of formula I; in the sodium salt, the molar ratio of the compound of formula I to sodium is 1:1.

7. The pharmaceutically acceptable salt of the compound of formula I according to claim 6, wherein the salt is a crystal form;
preferably, the crystal form is maleate crystal form A, and the maleate crystal form A has an X-ray powder diffraction pattern comprising diffraction peaks at 2θ±0.2° diffraction angles of 8.39°, 13.78°, 16.31°, 17.07°, and 18.44°; further, the maleate crystal form A has an X-ray powder diffraction pattern comprising diffraction peaks at 2θ±0.2° diffraction angles of 8.39°, 13.78°, 16.31°, 17.07°, 18.44°, 20.96°, 25.62°, and 26.78°; still further, the maleate crystal form A has an X-ray powder diffraction pattern comprising diffraction peaks at 2θ±0.2° diffraction angles of 8.39°, 13.78°, 16.31°, 17.07°, 18.44°, 20.96°, 21.61°, 25.29°, 25.62°, and 26.78°; yet further, the maleate crystal form A has an X-ray powder diffraction pattern comprising diffraction peaks at 2θ±0.2° diffraction angles of 8.39°, 13.78°, 16.31°, 17.07°, 18.44°, 20.45°, 20.96°, 21.61°, 25.29°, 25.62°, 26.19°, and 26.78°; further, the maleate crystal form A has an X-ray powder diffraction pattern comprising diffraction peaks at 2θ±0.2° diffraction angles of 8.39°, 13.78°, 16.31°, 17.07°, 18.44°, 19.01°, 20.45°, 20.96°, 21.61°, 22.65°, 23.19°, 24.62°, 25.29°, 25.62°, 26.19°, 26.78°, 27.03°, 27.41°, 28.47°, 28.96°, 30.05°, 31.25°, 31.61°, and 33.91°; further, the maleate crystal form A has an X-ray powder diffraction pattern comprising diffraction peaks at 2θ±0.2° diffraction angles of 8.39°, 10.65°, 13.78°, 16.31°, 17.07°, 18.44°, 19.01°, 20.45°, 20.96°, 21.61°, 22.65°, 23.19°, 24.62°, 25.29°, 25.62°, 26.19°, 26.78°, 27.03°, 27.41°, 28.47°, 28.96°, 30.05, 30.74°, 31.25°, 31.61°, 32.70°, 33.13°, 33.91°, 34.49°, 35.22°, 36.21°, and 38.48°; further, the maleate crystal form A has an XRPD pattern substantially as shown in FIG. 3-1;
preferably, the crystal form A of the maleate has one or two of the following characteristics:
(1) the TGA curve of the crystal form A of the maleate shows a weight loss of 0.5-3% at 120 ±3°C, preferably 0.5%, 1.0%, 1.5%, 2.0%, 2.5%, or 3.0%, for example 1.41%; and
(2) the crystal form A of the maleate has an endothermic peak at 139.1±3°C;
preferably, a TGA/DSC profile of the crystal form A of the maleate is substantially as shown in FIG. 3-2; and a ¹H NMR spectrum of the crystal form A of the maleate is substantially as shown in FIG. 3-3;
or the crystal form is crystal form A of the sodium salt of the compound of Formula I, wherein the crystal form A of the sodium salt has an X-ray powder diffraction pattern comprising diffraction peaks at 2θ±0.2° diffraction angles of 16.70°, 17.02°, 21.23°, 22.33°, 24.39°, 25.50°, and 25.89°; further, the crystal form A of the sodium salt has an X-ray powder diffraction pattern comprising diffraction peaks at 2θ±0.2° diffraction angles of 16.70°, 17.02°, 17.67°, 18.51°, 21.23°, 22.33°, 24.39°, 25.50°, 25.89°, and 29.73°; still further, the crystal form A of the sodium salt has an X-ray powder diffraction pattern comprising diffraction peaks at 2θ±0.2° diffraction angles of 16.70°, 17.02°, 17.67°, 18.51°, 21.23°, 22.33°, 24.39°, 25.50°, 25.89°, 29.73°, and 30.76°; yet further, the crystal form A of the sodium salt has an X-ray powder diffraction pattern comprising diffraction peaks at 2θ±0.2° diffraction angles of 4.85°, 9.70°, 12.13°, 13.55°, 14.20°, 14.55°, 16.70°, 17.02°, 17.67°, 18.51°, 21.23°, 22.33°, 24.39°, 25.50°, 25.89°, 26.72°, 27.74°, 28.44°, 29.73°, and 30.76°; further, the crystal form A of the sodium salt has an X-ray powder diffraction pattern comprising diffraction peaks at 2θ±0.2° diffraction angles of 4.85°, 9.70°, 12.13°, 13.55°, 14.20°, 14.55°, 16.70°, 17.02°, 17.67°, 18.51°, 20.27°, 21.23°, 22.33°, 23.35°, 24.39°, 25.50°, 25.89°, 26.72°, 27.74°, 28.44°, 29.73°, 30.76°, 31.43°, 31.88°, 32.46°, and 39.71°; still further, the crystal form A of the sodium salt has an X-ray powder diffraction pattern comprising diffraction peaks at 2θ±0.2° diffraction angles of 4.85°, 9.70°, 12.13°, 13.55°, 14.20°, 14.55°, 16.70°, 17.02°, 17.67°, 18.51°, 20.27°, 21.23°, 22.33°, 23.35°, 24.39°, 25.50°, 25.89°, 26.72°, 27.74°, 28.44°, 28.87°, 29.73°, 30.76°, 31.43°, 31.88°, 32.46°, 32.98°, 34.06°, 35.13°, 35.68°, 36.33°, 38.30°, and 39.71°; further, the crystal form A of the sodium salt has an XRPD pattern substantially as shown in FIG. 4-1;
preferably, the sodium salt crystal form A has one or two of the following characteristics:
(1) the TGA curve of the sodium salt crystal form A shows a weight loss of 1.0-5.0% at 150 ±3°C, preferably 1.0%, 1.5%, 2.0%, 2.5%, 3.0%, 3.5%, 4.0%, 4.5%, or 5.0%, for example, 2.75%; and
(2) the sodium salt crystal form A has two endothermic peaks at 191.0 ±3°C and 215.0 ±3°C;
preferably, a TGA/DSC profile of the sodium salt crystal form A is substantially as shown in FIG. 4-2; and a ¹H NMR spectrum of the sodium salt crystal form A is substantially as shown in FIG. 4-3.

8. A method for preparing a pharmaceutically acceptable salt of the compound of formula I according to claim 6, comprising mixing the compound of formula I with a suitable acid or base to obtain the pharmaceutically acceptable salt;
preferably, when the pharmaceutically acceptable salt of the compound of formula I is a maleate or a sodium salt, the preparation method comprises the following steps: mixing the compound of formula I with maleic acid or sodium hydroxide in an organic solvent A, stirring, and isolating the resulting solid; preferably, the stirring time is 1-5 days, and the stirring temperature is room temperature; the organic solvent A is selected from one or more of isopropanol, ethyl acetate, 2-methyltetrahydrofuran; preferably, mixing the compound of formula I with maleic acid or sodium hydroxide to obtain a suspension in the organic solvent A, and stirring the suspension;
preferably, when the pharmaceutically acceptable salt of the compound of formula I is maleate crystal form A, the preparation method comprises the following steps: mixing the compound of formula I with an equimolar amount of maleic acid to obtain a suspension in an organic solvent A, stirring the suspension, and isolating the resulting solid; preferably, the suspension stirring time is 1-5 days, and the suspension stirring temperature is room temperature; the organic solvent A is ethyl acetate;
preferably, when the pharmaceutically acceptable salt of the compound of formula I is sodium salt crystal form A, the preparation method comprises the following steps: mixing the compound of formula I with an equimolar amount of sodium hydroxide to obtain a suspension in an organic solvent A, stirring the suspension, and isolating the resulting solid; in some embodiments, the suspension stirring time is 1-5 days, for example, 3 days, and the suspension stirring temperature is room temperature; the organic solvent A is selected from one or more of isopropanol, ethyl acetate, 2-methyltetrahydrofuran.

9. A pharmaceutical composition, wherein the pharmaceutical composition comprises one or more of the crystal forms of the compound of formula I, the pharmaceutically acceptable salt thereof, or the crystal form of the pharmaceutically acceptable salt thereof according to any one of claims 1-3 and 6-7;
preferably, the crystal form of the compound of formula I is a free crystal form of the compound of formula I;
preferably, the crystal form of the compound of formula I is free crystal form A of the compound of formula I;
preferably, the crystal form of the compound of formula I is free crystal form B of the compound of formula I;
preferably, the crystal form of the pharmaceutically acceptable salt of the compound of formula I is maleate crystal form A or sodium salt crystal form A.

10. Use of the crystal form of the compound of formula I according to any one of claims 1-3 and 6-7, or a pharmaceutically acceptable salt thereof, or a crystal form of a pharmaceutically acceptable salt thereof, or the pharmaceutical composition according to claim 9, in the preparation of a medicament;
preferably, the medicament is for treating and/or preventing a voltage-gated sodium ion channel-related disease;
preferably, the voltage-gated sodium ion channel-related disease is a Nav1.8-related disease;
preferably, the Nav1.8-related disease comprises: pain;
preferably, the pain comprises: acute pain, chronic pain, inflammatory pain, cancer pain, neuropathic pain, musculoskeletal pain, primary pain, intestinal pain pain, or idiopathic pain.
